# EUROPEAN PATENT SPECIFICATION

(11) **EP 3 170 894 B1**
(45) Date of publication and mention of the grant of the patent: **01.04.2020**
(21) Application number: 15821596.2
(22) Date of filing: 16.07.2015
(51) Int. Cl.: C12N 5/077, C12N 5/0735

(54) **NEW UNDIFFERENTIATED STEM CELL REMOVAL AND MYOCARDIAL PURIFICATION AND REFINEMENT CULTURE MEDIUM**
NEUE UNDIFFERENZIERTE STAMMZELLENENTFERNUNG UND MYOKARDIALE REINIGUNGS- UND VERFEINERUNGSKULTURMEDIUM
NOUVELLE ÉLIMINATION DE CELLULES SOUCHES NON DIFFÉRENCIÉES ET NOUVEAU MILIEU DE CULTURE DE PURIFICATION ET D'AFFINAGE DU MYOCARDE

(30) Priority: 16.07.2014 JP 2014146283
(43) Date of publication of application: 24.05.2017
(73) Proprietor: Heartseed Inc., Tokyo 1500001 (JP)
(72) Inventor: FUKUDA, Keiichi, Tokyo 160-8582 (JP); FUJITA, Jun, Tokyo 160-8582 (JP); TOHYAMA, Shugo, Tokyo 160-8582 (JP)
(74) Representative: Vossius & Partner Patentanwälte Rechtsanwälte mbB
(86) International application number: PCT/JP2015/071048
(87) International publication number: WO 2016/010165

(56) References cited:
- WO-A1-2015/148515
- WO-A2-03/106661
- JP-A- 2011 500 009
- JP-A- 2013 143 968
- JUAN A. HERNÁNDEZ BORT ET AL: "CHO-K1 host cells adapted to growth in glutamine-free medium by FACS-assisted evolution", BIOTECHNOLOGY JOURNAL, vol. 5, no. 10, 1 October 2010 (2010-10-01), pages 1090-1097, XP055433300, DE ISSN: 1860-6768, DOI: 10.1002/biot.201000095
- ALTAMIRANO C ET AL: "Improvement of CHO cell culture medium formulation: simultaneous substitution of glucose and glutamine", BIOTECHNOLOGY PROGRESS, AMERICAN INSTITUTE OF CHEMICAL ENGINEERS, US, vol. 16, 1 January 2000 (2000-01-01), pages 69-75, XP002201424, ISSN: 8756-7938, DOI: 10.1021/BP990124J
- CORINNA SELIGER ET AL: "Lactate-Modulated Induction of THBS-1 Activates Transforming Growth Factor (TGF)-beta2 and Migration of Glioma Cells In Vitro", PLOS ONE, vol. 8, no. 11, 1 November 2013 (2013-11-01), page e78935, XP055433309, DOI: 10.1371/journal.pone.0078935
- SHUGO TOHYAMA ET AL: "Distinct Metabolic Flow Enables Large-Scale Purification of Mouse and Human Pluripotent Stem Cell-Derived Cardiomyocytes", CELL STEM CELL, vol. 12, no. 1, 1 January 2013 (2013-01-01), pages 127-137, XP055175411, ISSN: 1934-5909, DOI: 10.1016/j.stem.2012.09.013
- PAUL W BURRIDGE ET AL: "Chemically defined generation of human cardiomyocytes", NATURE METHODS, vol. 11, no. 8, 15 July 2014 (2014-07-15), pages 855-860, XP055239845, ISSN: 1548-7091, DOI: 10.1038/nmeth.2999
- SHERWIN TING: "Nutrient supplemented serum-free medium increases cardiomyogenesis efficiency of human pluripotent stem cells", WORLD JOURNAL OF STEM CELLS, vol. 5, no. 3, 1 January 2013 (2013-01-01) , page 86, XP055130446, ISSN: 1948-0210, DOI: 10.4252/wjsc.v5.i3.86
- NOBUAKI SHIRAKI ET AL: "Methionine Metabolism Regulates Maintenance and Differentiation of Human Pluripotent Stem Cells", CELL METABOLISM, vol. 19, no. 5, 1 May 2014 (2014-05-01), pages 780-794, XP055220770, United States ISSN: 1550-4131, DOI: 10.1016/j.cmet.2014.03.017
- TOHYAMA SHUGO ET AL: "Glutamine Oxidation Is Indispensable for Survival of Human Pluripotent Stem Cells", CELL METABOLISM, CELL PRESS, UNITED STATES, vol. 23, no. 4, 31 March 2016 (2016-03-31) , pages 663-674, XP029504671, ISSN: 1550-4131, DOI: 10.1016/J.CMET.2016.03.001
- YAMAMOTO K. ET AL.: 'The Production of Urea from Ornithine in Rat Hepatoma Cells Continuously Cultured in a Chemically Defined Medium' CELL STRUCTURE AND FUNCTION vol. 6, no. 4, 1981, pages 367 - 74, XP003032449
- WANG J. ET AL.: 'Dependence of mouse embryonic stem cells on threonine catabolism' SCIENCE vol. 325, no. 5939, 24 July 2009, pages 435 - 9, XP055102216
- TOHYAMA S. ET AL.: 'Distinct metabolic flow enables large-scale purification of mouse and human pluripotent stem cell -derived cardiomyocytes' CELL STEM CELL vol. 12, no. 1, 03 January 2013, pages 127 - 37, XP055175411
- KEIICHI FUKUDA: 'Clinical application of iPS cells in cardiovascular field' THE JOURNAL OF THE JAPANESE SOCIETY OF INTERNAL MEDICINE vol. 102, no. 9, 10 September 2013, pages 2232 - 2240, XP008185486
- SHIRAKI N. ET AL.: 'Methionine metabolism regulates maintenance and differentiation of human pluripotent stem cells' CELL METAB. vol. 19, no. 5, 06 May 2014, pages 780 - 94, XP055220770

## Description

### TECHNICAL FIELD

The present invention relates to provision of culture media that can be used for elimination of undifferentiated stem cells (SCs) and purification and refinement of cardiomyocytes (CMs).

### BACKGROUND ART

In adult individuals, cardiomyocytes lack proliferative activity, so in order to treat serious myocardial diseases such as myocardial infarction and cardiomyopathy, there has conventionally been no choice but to rely on cardiac transplantation. However, as studies have progressed in recent years on pluripotent stem cells (PSCs) such as embryonic stem cells (ESCs) and induced pluripotent stem cells (iPSCs), it is becoming possible to induce the differentiation of such PSCs to prepare CMs, and to use such induced CMs in medical transplantation.

In this connection, however, cardiomyocyte differentiation under unphysiological conditions (*i.e., in vitro* conditions) takes place in a similar manner to the case of physiological development of CMs: undifferentiated mesodermal cells are first formed and then the undifferentiated mesodermal cells differentiate -- some of them differentiate through presumptive cardiomyocytes (precardiac mesoderm) -- into CMs. Moreover, PSCs are essentially capable of differentiating into all types of cells constituting organs. For these reasons, it is technically difficult to allow PSCs to differentiate into a single limited type of cells, *i.e.* CMs only, by simply inducing the differentiation of the PSCs. Also, under unphysiological conditions (*i.e., in vitro* conditions), it is difficult to elicit the intended differentiation of all PSCs, and some PSCs may remain undifferentiated after the induction of differentiation.

Thus, when SCs are induced *in vitro* to differentiate into CMs, there remain various detrimental factors to clinical applications including transplantation: for example, PSCs all have the potential to produce as a byproduct other types of cells than CMs, and some PSCs may remain undifferentiated. In particular, residual undifferentiated SCs have proliferative activity and are capable of differentiating into a wide variety of cells; thus, if any undifferentiated SCs remain in cells transplanted into the body for treatment, there is a significantly high risk that teratomas may be formed from the undifferentiated SCs (Non-Patent Literature 1 (Miura, et al., Nature Biotech., 743-745, 2009)). For these reasons, CM-containing cell masses prepared by inducing the differentiation of PSCs are difficult to transplant into the body as they are and to use for therapeutic purposes. Therefore, in order to safely conduct medical therapies with the use of CMs derived from PSCs and to obtain ideal efficacy from those therapies, it is necessary to discover a method for completely eliminating undifferentiated SCs and highly purifying CMs (*i.e.,* a method for eliminating other types of cells than CMs).

There have hitherto been reported some methods for selectively eliminating undifferentiated SCs under culture conditions (Non-Patent Literature 2 (Ben-David, et a/., Cell Stem Cell, 2013 12, pp. 167-179), Non-Patent Literature 3 (Wang, et al., Science, 325, 435-439, 2009)), and Non-Patent Literature 4 (Shiraki, et al., Cell Metabolism, 2014, 19, 1-15). In Non-Patent Literature 2, it was found that oleate is important to maintain human undifferentiated PSCs, and that with the aid of this requirement to the contrary, human undifferentiated PSCs can be selectively eliminated by inhibiting oleate biosynthesis in human undifferentiated PSCs. In Non-Patent Literature 3, it was found that murine ESCs can colonize in culture media individually deprived of each of other amino acids than threonine but cannot colonize in a culture medium deprived of threonine, and that murine undifferentiated ESCs can be selectively eliminated by culturing them in a culture medium deprived of threonine. In Non-Patent Literature 4, it was found that methionine plays the same role in human PSCs as that played by threonine in murine ESCs, and that human undifferentiated SCs can be selectively eliminated in a culture medium deprived of methionine, in which undifferentiated SCs undergo cell death or differentiate.

However, the methods discovered in these literatures allow elimination of undifferentiated PSCs but do not allow elimination of non-cardiomyocytes (non-CMs) which are produced through induction of the differentiation of PSCs into other types of cells than CMs. Further, the methods of Non-Patent Literatures 3 and 4 eliminate the certain essential amino acids which are very important for protein synthesis, so there is a concern that the absence of those amino acids may have an influence on the intended surviving cells.

Thus, the present inventors' study group has focused not on the essential amino acids which are very important for protein synthesis but on nonessential amino acids which may be synthesized or supplied from other sources, and have made studies with a view to efficiently eliminating not only undifferentiated SCs which remain without being induced to differentiate when PSCs are induced to differentiate into CMs, but also non-CMs which are produced as a byproduct of the induction of CM differentiation. As a result, the inventors' group found out different physiological characteristics which are possessed by CMs but not by other types of cells, and developed a method for selecting CMs only from non-CMs and undifferentiated SCs through the use of those physiological characteristics.

Patent Literature 1: A method of selecting CMs from a CM-containing cell mixture without genetic alteration of the CMs on the basis of a relative content of cellular mitochondria and/or a relative mitochondrial transmembrane potential of the cell; a method of enriching CMs from a CM-containing cell mixture without genetic alteration of the CMs; a method of producing CMs without genetic alteration of CMs; and a method of evaluating ratio of CMs in a CM-containing cell mixture (WO 2006/022377);

Patent Literature 2: CMs derived from ESCs can be efficiently and highly selected and purified by culturing the CMs in a culture medium under the following conditions: a low-serum-supplemented condition, a low-glucose-supplemented condition, a low-nutritional condition, a low calcium condition, a mildly-acidic pH condition, a lactate-supplemented condition, an aspartate/glutamate-supplemented condition, and/or a pyruvate-supplemented condition (WO 2007/088874);

Patent Literature 3: A method of preparing cell masses of CMs derived from PSCs, characterized in that purified CMs derived from PSCs obtained by dissociating aggregated cell masses containing CMs differentiated and induced from PSCs into single cells are cultured in a culture medium under a serum-free condition whereby the purified CMs are reaggregated (WO 2009/017254);

Patent Literature 4: A method for extremely efficiently inducing cell death of other cells than CMs by adding a substance that is not recognized to have physical toxicity or cell death-inducing activity to the culture conditions for PSCs or non-CMs (WO 2010/114136); and

Patent Literature 5: A method for measuring the activity potential of cultured CMs, which comprises bringing a potential-sensitive fluorochrome into contact with CMs being cultured in a culture medium, adding vitamin E and/or cholesterol to the culture medium, and measuring changes in fluorescent intensity of the potential-sensitive fluorochrome depending on potential or ionic strength changes (WO 2011/052801).

Notably, in Patent Literature 2, it was found that CMs are highly tolerant of culture under the conditions that are generally considered as severe cell culture conditions (*e.g*., a low-glucose-supplemented condition, a low-serum-supplemented condition, a mildly-acidic pH condition, a low calcium condition, a low-nutritional condition, a lactate-supplemented condition, an aspartate/glutamate-supplemented condition, and/or a pyruvate-supplemented condition), and that under such conditions, only CMs survive and other types of cells than CMs (*i.e.,* non-CMs and undifferentiated SCs) undergo cell death, and consequently CMs can be selected. Further, the study in murine PSCs showed that the use of particularly a low-glucose-supplemented condition and a lactate-supplemented condition among the aforementioned conditions most efficiently contributes to selection of CMs, and thus, investigation was made to determine whether a low-glucose-supplemented condition and a lactate-supplemented condition can be used for the purpose of selecting CMs derived from human PSCs (hPSCs).

However, the study on CMs derived from hPSCs revealed that under the low-glucose-supplemented culture condition and the lactate-supplemented culture condition, it is possible to eliminate undifferentiated SCs and pick up CMs, but that it takes time to eliminate undifferentiated SCs and that in clinical applications using hundreds of millions of cells, undifferentiated SCs are highly likely to be mixed in CMs. Accordingly, there has been a need for a system that can eliminate undifferentiated SCs more completely and in a shorter time.

Cells acquire a major part of energy required for their survival by producing ATP through different mechanisms based on the catabolic reaction of glucose. The pathways up to the production of ATP based on the catabolic reaction of glucose are known to be mainly composed of: the glycolytic system which is a process of degrading glucose incorporated in cells into pyruvate; the TCA cycle (citric acid cycle) which operates with acetyl-CoA generated by degradation of pyruvate; and the electron transport system which produces APT from NADH, NADPH, and FADH₂ generated in the glycolytic system and the TCA cycle.

In general, cells acquire energy based on the precondition that they take in glucose from the environment. When cells acquire energy on that basis, the glycolytic system is activated, so that after glucose is degraded in the glycolytic system, energy is acquired through the TCA cycle (citric acid cycle) and electron transport system. When energy is needed rapidly, energy is obtained by anaerobic respiration with the full use of the glycolytic system; thus, the production of pyruvate surpasses its consumption, thereby leading to activation of the lactate fermentation pathway. In contrast, the reverse reaction for converting lactate back to pyruvate is relatively not activated.

### CITATION LIST

### PATENT LITERATURES

[Patent Literature 1] WO 2006/022377
[Patent Literature 2] WO 2007/088874
[Patent Literature 3] WO 2009/017254
[Patent Literature 4] WO 2010/114136
[Patent Literature 5] WO 2011/052801

### NON-PATENT LITERATURES

[Non-Patent Literature 1] Miura et al., Nature Biotech., 743-745, 2009
[Non-Patent Literature 2] Ben-David, et al., Cell Stem Cell, 2013 12, pp. 167-179
[Non-Patent Literature 3] Wang, et al., Science, 325, 435-439, 2009
[Non-Patent Literature 4] Shiraki, et al., Cell Metabolism, 2014, 19, 1-15

### SUMMARY OF INVENTION

### TECHNICAL PROBLEM

As mentioned above, when cell culture is performed under the low-glucose-supplemented condition and the lactate-supplemented condition as defined in Patent Literature 2, surviving non-CMs or undifferentiated SCs may be present in small numbers depending on the condition. Therefore, there existed a need to find out new conditions that make it possible to eliminate undifferentiated SCs in a shorter time and, consequently, to induce cell death of non-CMs or undifferentiated SCs more completely and select CMs only.

### SOLUTION TO PROBLEM

In order to achieve the aforementioned objects, there are provided in the present application the embodiments as characterized in the claims. Thus, the present invention relates to the following items:
1. Use of a cell culture medium which has less than 10% of glutamine in the amino acid profile for inducing cell death of undifferentiated stem cells.
2. A method for inducing cell death of undifferentiated stem cells by performing cell culture in a cell culture medium that has less than 10 % of glutamine in the amino acid profile.
3. The use according to item 1 or the method according to item 2, wherein the undifferentiated stem cells are pluripotent stem cells or multipotent stem cells.
4. The use according to item 1 or 3 or the method according to item 2 or 3, wherein the cell culture medium is further free of serine and glycine in the amino acid profile.
5. The use according to any one of items 1, 3 and 4 and 5 or the method according to any one of items 2-4, wherein the cell culture medium is further free of arginine in the amino acid profile.
6. The method according to any one of items 2-5, wherein the cell culture is continued for 12-360 hours under any of the above particular amino acid-free conditions.
7. Use of a cell culture medium which is:
   supplemented with lactate, free of sugar, and has less than 10 % of glutamine in the amino acid profile; or
   supplemented with pyruvate, free of sugar, and has less than 10 % of glutamine in the amino acid profile; or
   supplemented with a fatty acid, free of sugar, and has less than 10 % of glutamine in the amino acid profile,
   for selecting cardiomyocytes from a mixture of cardiomyocytes and non-cardiomyocytes.
8. The use according to item 7, wherein the non-cardiomyocytes are cells including undifferentiated stem cells, other differentiated cells than cardiomyocytes, and established cells.
9. A method for selecting cardiomyocytes, comprising:
   culturing a mixture of cardiomyocytes and non-cardiomyocytes in a cell culture medium which is supplemented with lactate, free of sugar, and has less than 10 % of glutamine in the amino acid profile; or
   culturing a mixture of cardiomyocytes and non-cardiomyocytes in a cell culture medium which is supplemented with pyruvate, free of sugar, and has less than 10 % of glutamine in the amino acid profile; or
   culturing a mixture of cardiomyocytes and non-cardiomyocytes in a cell culture medium which is supplemented with a fatty acid, free of sugar, and and has less than 10 % of glutamine in the amino acid profile.
10. The method according to item 9, wherein the non-cardiomyocytes are undifferentiated stem cells, differentiated non-cardiomyocytes, or established cells.
11. The use according to item 7 or the method according to item 9 or 10, wherein the cell culture medium is further free of serine and glycine in the amino acid profile.
12. The use according to item 7 or 11 or the method according to any one of items 9-11, wherein the cell culture medium is further free of arginine in the amino acid profile.
13. The method according to any one of items 9-12, wherein the cell culture is continued for 12-360 hours under any of the above particular amino acid-free conditions.
14. The use according to any one of items 7, 8, 11 and 12 or the method according to any one of items 9-13, wherein the cell culture medium is supplemented with ascorbate or albumin.

Moreover, in accordance with the above, there is disclosed the following:
a cell culture medium for use in inducing cell death of undifferentiated SCs, wherein the cell culture medium is free of glutamine in the amino acid profile; and also a method for inducing cell death of undifferentiated SCs by performing cell culture in said cell culture medium. Further disclosed are: a cell culture medium for use in selecting CMs, wherein the cell culture medium is supplemented with a fatty acid, lactate or pyruvate, free of sugar, and free of glutamine in the amino acid profile; and also a method for selecting CMs by culturing a mixture of CMs and non-CMs in said cell culture medium.

### ADVANTAGEOUS EFFECTS OF INVENTION

According to a first aspect, there is disclosed a cell culture medium for use in inducing cell death of undifferentiated SCs; and simply by performing cell culture using said cell culture medium, undifferentiated SCs can be easily induced to undergo cell death. Also, according to the second aspect , there is disclosed a cell culture medium for use in selecting CMs; and simply by performing cell culture using said cell culture medium, not only undifferentiated SCs, such as PSCs including human ESCs (hESCs) and human iPSCs (hiPSCs), but also other differentiated cells than CMs, and established cells, as defined below, can be easily induced to undergo cell death, and consequently, CMs can be selected.

### BRIEF DESCRIPTIONS OF DRAWINGS

FIG. 1 shows change in the concentrations in medium of amino acids and glucose consumed when 2.5×10⁵ hESCs are cultured for 3 days. The concentrations of amino acids and glucose at day 0 before exposed to the cells were expressed as 100%.
FIG. 2 shows the results of observing the alkaline phosphatase (ALP) staining of hESCs cultured under different conditions (with/without glucose, with/without lactate, and without any one of the different nonessential amino acids which were highly consumed as per FIG. 1).
FIG. 3 shows the results of observing the survival or death of hESCs cultured under different conditions (with/without glucose, with/without lactate, and without any one of the different nonessential amino acids which were highly consumed as per FIG. 1).
FIG. 4 shows the results of observing the alkaline phosphatase (ALP) staining of hiPSCs cultured under different conditions (with/without glucose, with/without lactate, and without any one of the different nonessential amino acids which were highly consumed as per FIG. 1).
FIG. 5 shows the results of observing the alkaline phosphatase (ALP) staining of hESCs cultured under different conditions that are free of glucose and also free of any one of different amino acids.
FIG. 6 shows the results of observing the survival or death of murine neonatal CMs cultured under different conditions (with/without glucose, with/without lactate, and without any one of the different nonessential amino acids which were highly consumed as per FIG. 1).
FIG. 7 shows the photos of cell masses that were obtained by inducing two-dimensional CM differentiation from hiPSCs and then cultured in a lactate-supplemented, glucose-free medium (Gluc⁻, All⁺, Lactate⁺) or a lactate-supplemented, glucose- and glutamine-free medium (Gluc⁻, Gln⁻, Lactate⁺).
FIG. 8 shows that when the cell masses obtained in FIG. 7 were dissociated with 0.25% Trypsin EDTA and cultured in a fibronectin-coated culture dish, only CMs survived. B: Most of α-Actinin-positive CMs were positive for Troponin I. C: It was confirmed that after refinement and purification using the lactate-supplemented, glucose- and glutamine-free medium (Gluc⁻, Gln⁻, Lactate⁺), no undifferentiated SCs positive for Tral-60 survived at all.
FIG. 9 shows the results of QPCR analysis of residual undifferentiated SCs after induction of CM differentiation or after refinement and purification. Lin28 was detected after refinement and purification using the glutamine-supplemented, lactate-supplemented, glucose-free medium (Gluc⁻, All⁺, Lactate⁺), whereas no Lin28 was detected at all after refinement and purification using the lactate-supplemented, glucose- and glutamine-free medium (Gluc⁻, Gln⁻, Lactate⁺).

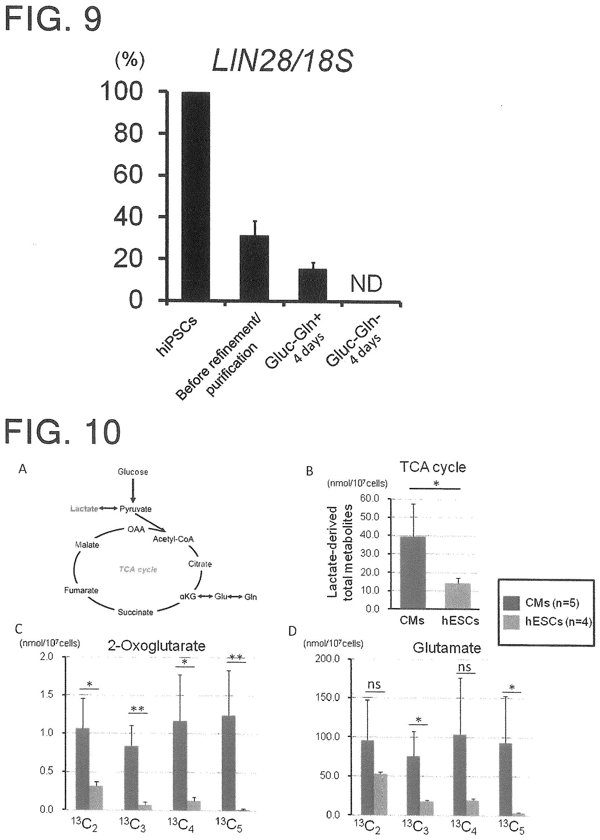

FIG. 10 shows the difference in lactate metabolism between murine neonatal CMs and hESCs under a glucose- and glutamine-free culture condition. By treating the cell samples with a culture medium supplemented with ¹³C-labeled lactate and quantifying them for metabolites in the TCA cycle and glutamate biosynthesis, it can be analyzed how lactate is metabolized. FIG. 10A shows a schematic map of lactate-related metabolism. It was found that the total amount of lactate-derived metabolites in the TCA cycle was significantly high in CMs. Also, ¹³C labels were detected in significantly larger amounts in 2-oxoglutarate and glutamate in CMs. The above data show that in CMs, lactate more greatly constributes to the biosynthesis of glutamate. *p<0.05, **p<0.01.
FIG. 11 shows the results of FACS analysis of the percentage of Troponin T-positive cells in cell groups obtained after induction of differentiation of hiPSCs.
FIG. 12 shows the results of observing the alkaline phosphatase (ALP) staining of hESCs cultured under different conditions (without glucose, with/without glutamine, with/without α-ketoglutarate, and with/without pyruvate).
FIG. 13 shows the results of observing the survival or death of murine neonatal CMs cultured under different conditions (without glucose, with/without glutamine, with/without pyruvate, and with/without lactate).

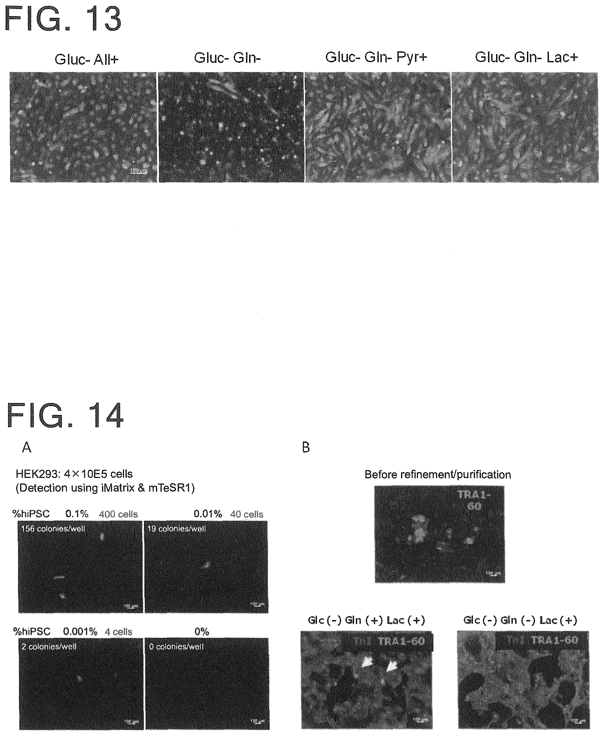

FIG. 14A shows the results of evaluating the detection sensitivity of a detection method for residual undifferentiated SCs (Tano, et al., PLOS ONE, 2014). FIG. 14B shows the results of evaluating the presence of residual undifferentiated SCs after induction of CM differentiation or after refinement and purification by following the method tested in FIG. 14A. TRA1-60, a detection marker for undifferentiated SCs, was detected after refinement and purification using a lactate-supplemented, glutamine-supplemented, glucose-free medium (Gluc⁻, Gln⁺, Lac⁺), whereas no TRA1-60 was detected after refinement and purification using a lactate-supplemented, glucose- and glutamine-free medium (Gluc⁻, Gln⁻, Lactate⁺).
FIG. 15 shows the survival or death of hESCs-derived (proliferative) non-CMs cultured under each of a glucose-free, glutamine-supplemented condition and a glucose- and glutamine-free, lactate-supplemented condition.
FIG. 16 shows the results of observing different groups of hiPSCs cultured in each of lactate-supplemented, glucose- and glutamine-free media (Gluc⁻, Gln⁻, Lac⁺) which were further supplemented with ascorbate (25 mg/L) or albumin (0.1%).
FIG. 17 shows the results of observing different groups of hiPSCs-derived non-CMs cultured in each of lactate-supplemented, glucose- and glutamine-free media (Gluc⁻, Gln⁻, Lac⁺) which were further supplemented with ascorbate (25 mg/L) or albumin (0.1%).
FIG. 18 shows the results of observing different groups of hiPSCs-derived CMs cultured in each of lactate-supplemented, glucose- and glutamine-free media (Gluc⁻, Gln⁻, Lac⁺) which were further supplemented with ascorbate (25 mg/L) or albumin (0.1%).
FIG. 19 shows the results of observing different groups of hiPSCs-derived CMs cultured for 744 hours in each of lactate-supplemented, glucose- and glutamine-free media (Gluc⁻, Gln⁻, Lac⁺) which were further supplemented with ascorbate (25 mg/L) or albumin (0.1%).

### DESCRIPTION OF EMBODIMENTS

The glycolytic system is a fundamental system for energy acquisition which is found in most organisms, and a metabolic pathway in which glucose is anaerobically degraded into pyruvate or lactate. In the body of animals, glucose is phosphorylated by the γ-phosphate group of ATP, or glycogenolysis is activated, to produce glucose-6-phosphate, as a first reaction. Then, the glucose-6-phosphate is successively metabolized to fructose-1,6-bisphosphate, which is cleaved to triose phosphate, and then the triose phosphate is converted to pyruvate while ATP is generated. During this process, 2 mol of ATP is produced, 2 mol of NAD⁺ is reduced, and 2 mol of NADH is produced, in total, per mol of glucose. The final product of this metabolic pathway, pyruvate, is converted into lactate and released out of cells, or is transported into mitochondria, acting as a substrate of the citric acid cycle.

The tricarboxylic acid cycle (TCA cycle or citric acid cycle) is a metabolic pathway for finally oxidizing completely the carbon skeletons of sugars, fatty acids, many amino acids and the like. For example, looking at the glycolytic system, the final product of the glycolytic system, pyruvate, is degraded to generate acetyl-CoA, and the acetyl-CoA is condensed with oxaloacetate to generate citrate. Then, the citrate is converted successively (via cis-aconitate) into isocitrate, which undergoes dehydrodecarboxylation to give 2-oxoglutarate. Further, the 2-oxoglutarate successively undergoes dehydrodecarboxylation, CoA elimination, dehydrogenation, hydration, dehydrogenation, and other reactions to become succinyl-CoA, succinate, fumarate, malate, and finally oxaloacetate. In one turn of the TCA cycle, the acetyl groups in acetyl-CoA are completely oxidized; and in the degradation of one molecule of acetyl-CoA, two molecules of CO₂ are released, three molecules of NADH are produced, one molecule of reduced FAD is produced, and one molecule of GTP is produced.

However, the TCA cycle is not merely a degradation process but is a regulatory point for switching between the metabolic systems of sugars, amino acids and fatty acids, serving as an important starting point for assimilatory metabolism. For example, when glycolysis proceeds to some extent, the concentrations of citrate, etc. become high and acetyl-CoA carboxylase is activated, so that the operation of the TCA cycle itself is suppressed within a certain limit and that acetyl-CoA is diverted to the synthesis of fatty acids. Also, for example, oxaloacetate is transformed into aspartate. Thus, since TCA cycle intermediates are shunt away for the biosynthesis of various organic substances, the amount of oxaloacetate produced in one turn of the TCA cycle commonly decreases. Therefore, in order to maintain smooth operation of the TCA cycle, separate systems for replenishing oxaloacetate are required; and examples of such a system include: (along with systems for degrading each of alanine, glycine, cysteine, serine, and threonine into pyruvate) a system for degrading pyruvate by pyruvate carboxylase to supply oxaloacetate; a system for transaminating aspartate by aspartate transaminase to generate oxaloacetate; a system for degrading phenylalanine and tyrosine to generate fumarate; and a system for degrading arginine, glutamine, histidine and proline into glutamate and then oxidizing glutamate by glutamate dehydrogenase to supply 2-oxoglutarate.

In energy acquisition systems in animal cells, hydrogen atoms and electrons generated by different dehydrogenation reactions in the glycolytic system and the TCA cycle as described above are transported into mitochondria by an electron donor such as NADH, NADPH or FADH₂, and passed to a series of redox enzymes, *i.e.* oxidoreductases (complexes), and electron carriers (e.g., cytochromes) found in the inner mitochondrial membrane. The enzymes are reduced by the electron carrier carrying hydrogen atoms and electrons to generate water. In this process, H⁺ ions are unidirectionally transported across the membrane to form an electrochemical potential difference of H⁺ ions, and finally, high-energy phosphate bonds as found in ATP are synthesized using this electrochemical potential difference.

Since when cell culture is performed under the known different culture conditions as defined in Patent Literature 2, surviving non-CMs or undifferentiated SCs may be present in small numbers, the present inventors have made intensive studies with a view to finding out new conditions that make it possible to induce cell death of non-CMs or undifferentiated SCs more completely and in a shorter time and to select CMs only.

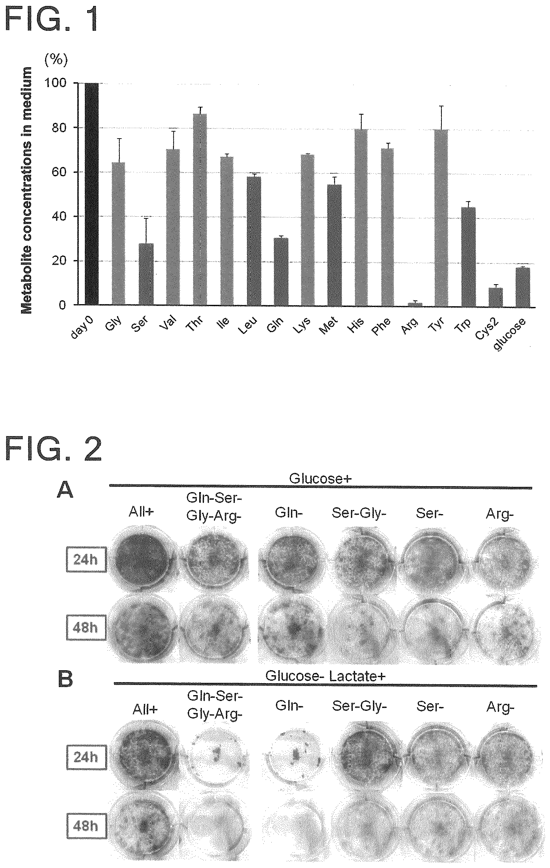

First, the inventors investigated amino acids actively consumed in hPSCs by measuring the amino acid concentrations in a fresh culture medium and in a culture medium after use for cell culture. As a result, it was found that the consumptions of serine (Ser), glutamine (Gln), arginine (Arg) and cystine (Cys2) are, in general, high in hPSCs. It was also found that the consumptions of leucine (Leu), methionine (Met) and tryptophan (Trp) are relatively high (FIG. 1). However, since there is a concern that the absence of essential amino acids which are very important for protein synthesis, as in the cases of Non-Patent Literatures 3 and 4, may have an influence on the intended surviving cells, the inventors made further studies focusing on nonessential amino acids which may be synthesized or supplied from other sources.

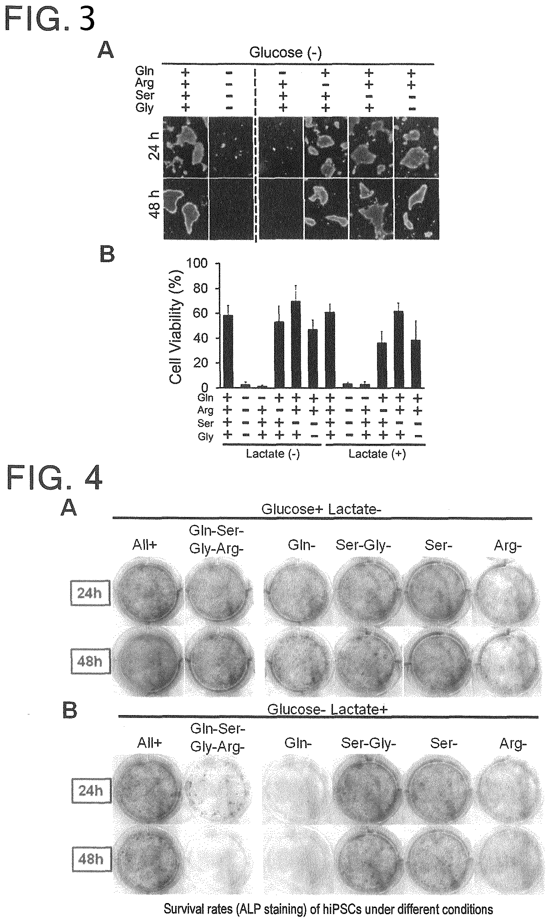

As a result of culturing hESCs or hiPSCs in the presence of glucose under different amino acid conditions, it was found that when hESCs or hiPSCs are cultured in cell culture media that are supplemented with glucose (Glue) but are free of serine (Ser), glycine (Gly), glutamine (Gln), or arginine (Arg), the number of hESC or hiPSC colonies decreases (FIGs. 2A and 4A). Further, the analysis of the effect of those amino acid-free culture conditions on the number of hESC/hiPSC colonies showed that under a glucose-free culture condition, glutamine (Gln), serine (Ser) & glycine (Gly), and arginine (Arg), in this order, exhibited the most potent effect (FIG. 3B).

On the basis of these results, the present inventors found that according to the present invention, by performing cell culture in a culture medium that is free of glutamine in the amino acid profile (Gln⁻), undifferentiated SCs, such as PSCs including hESCs and hiPSCs, and other differentiated cells than CMs as defined below, can be induced to undergo cell death; thus, the inventors have completed this invention.

Accordingly, in the first aspect , there is disclosed a cell culture medium that is free of glutamine in the amino acid profile (Gln⁻). On the basis of the aforementioned findings, the cell culture medium of the first aspect may be further characterized by being free of serine and/or glycine (Ser⁻, Gly⁻) and still further characterized by being free of arginine (Arg⁻). Specific examples of the cell culture medium that can be used include, but are not limited to, the following:
- a cell culture medium that is free of glutamine in the amino acid profile (Gln⁻);
- a cell culture medium that is free of glutamine and serine in the amino acid profile (Gln⁻, Ser);
- a cell culture medium that is free of glutamine and glycine in the amino acid profile (Gln⁻, Gly⁻);
- a cell culture medium that is free of glutamine, serine and glycine in the amino acid profile (Gln⁻, Ser⁻, Gly⁻);
- a cell culture medium that is free of glutamine and arginine in the amino acid profile (Gln⁻, Arg⁻);
- a cell culture medium that is free of glutamine, serine and arginine in the amino acid profile (Gln⁻, Ser⁻, Arg⁻);
- a cell culture medium that is free of glutamine, glycine and arginine in the amino acid profile (Gln⁻, Gly⁻, Arg⁻); and
- a cell culture medium that is free of glutamine, serine, glycine and arginine in the amino acid profile (Gln⁻, Ser⁻, Gly⁻, Arg⁻).

The aforementioned amino acids are all related to the sugar metabolism and TCA cycle as described above in the section titled "TECHNIAL FIELD". Glutamine is degraded into glutamate, which is then oxidized by glutamate dehydrogenase to 2-oxoglutarate and introduced into the TCA cycle. Serine and glycine are each degraded into pyruvate, which is then broken down by pyruvate carboxylase to give oxaloacetate and introduced into the TCA cycle.

The aforementioned inventive cell culture medium of the first aspect is a culture medium free of serum or a serum alternative -- other conditions for the medium, besides those of the particular amino acids (*i.e.,* glutamine, serine and/or glycine, or arginine), can be the same as those for common cell culture media (for example, Dulbecco's modified eagle's medium (DMEM), MEM culture media (*e.g*., α-MEM, MEM [Hank's BSS]), RPMI culture media (*e.g.*, RPMI 1640), F12 culture medium, StemPro34, and mTeSR1).

According to the studies made by the present inventors, the aforementioned cell culture media having unique amino acid profiles can be used to induce cell death of undifferentiated SCs, and other differentiated cells than CMs as defined below. However, in order to induce cell death of those cells more efficiently, a low-glucose condition (Gluc⁻) which is free of sugar can be adopted in addition to the aforementioned amino acid profile conditions.

As referred to herein, "undifferentiated stem cells (SCs)" refers to stem cells having pluripotency or multipotency which are commonly used in the technical field to which the present invention belongs, and includes ESCs, all other types of PSCs (*e.g.,* iPSCs) having similar characters to those of ESCs, and multipotent SCs as found in adult organ/tissue cells, bone marrow cells and blood cells of mammals. As referred to above, "similar characters to those of ESCs" can be defined by the cell biological characters specific to ESCs, such as the presence of surface (antigenic) markers specific to ESCs, the expression of ESC-specific genes, or teratoma-forming ability or chimeric mouse-forming ability. In this invention, it is preferred that the undifferentiated SCs be PSCs.

The undifferentiated SCs can also be defined by having their unique cell markers, such as OCT3/4, NANOG, TRA1-60, TRA1-81, SSEA-3 and SSEA-4.

As regards preparation of CMs from PSCs, it is considered that as the differentiation proceeds, PSCs are converted into undifferentiated mesoderm and then cardiac mesoderm (or presumptive cardiomyocytes) and thereafter differentiate into CMs. Therefore, "cardiomyocytes (CMs)", as referred to in this invention, is a concept that includes all types of cells generated during induction of the differentiation of undifferentiated SCs into CMs, and covers all of undifferentiated mesoderm, cardiac mesoderm (or presumptive cardiomyocytes), and subsequent cardiomyocytes as mentioned above. As referred to above, "undifferentiated mesoderm" refers to cells that are observed to express Brachyury proteins specific to undifferentiated mesoderm. "Cardiac mesoderm (or presumptive cardiomyocytes)" refers to cells that are observed to express proteins like Mesp-1 specific to the mesoderm initiated to differentiate into the heart but are still not observed to express CM-specific proteins like Nkx2.5 and Actinin, and which have a capability of exclusively differentiating into CMs without the need for any subsequent further induction. Also, "cardiomyocytes" means viable cells spontaneously beating, or immobilized cells expressing markers such as Nkx2.5, GATA4 and Actinin.

In fetal period, the blood fatty acid concentration is 0.1 mM or lower and the blood lactate concentration is in the range of 5-7 mM; thus, CMs use lactate as a main energy source (Tohyama S., et al., Cell Stem Cell., 2013;12:127-137). In postnatal period, the blood fatty acid concentration increases to 0.2-0.4 mM and the blood lactate concentration decreases to 0.5 mM; thus, CMs use fatty acids as a main energy source (Lopaschuk G.D., et al., Am J Physiol., 1991; 261:H1698-1705; Werner J.C., *et al.,* 1987;22:552-556; Medina J.M., Biol Neonate., 1985;48:237-244; Lopaschuk G.D., et al., J Cardiovasc Pharmacol., 2010;56:130-140). When postnatal CMs are stressed by ischemia, pressure overload, etc., their gene expression pattern changes to the fetal one, so that they come to be able to use lactate as a main energy source as fetal CMs do. In general, "cardiomyocytes (CMs)", unlike other types of cells, have a common characteristic in that they can use lactate, pyruvate and fatty acids instead of glucose as an energy source. And according to the present invention using such a characteristic shared in common by CMs only, and not by other types of cells, purification of CMs can be conducted. Therefore, the "CMs" used in this invention are not limited by their origin or the method for obtaining them. Examples of the CMs include, but are not limited to: "CMs" obtained by inducing the differentiation of PSCs; "CMs" harvested from human foetuses, neonates and adults; "CMs" harvested from foetuses, neonates and adults of animals belonging to mammals; and "CMs" obtained by direct reprogramming of differentiated non-CMs.

As referred to in the present invention, "established cells" refers to immortalized cells that are capable of self-replication under cell culture conditions.

In the present invention, when a cell culture medium is defined as being "free" of any of the particular amino acids (*i.e.,* serine, glycine, glutamine, or arginine) and/or sugar, it is ultimately desirable that the culture medium be completely free of those components, but to the extent that the object of the invention can be achieved, it is not necessarily required that the culture medium be deprived of 100% of those components, and it is acceptable even if any of these amino acids is present in the culture medium in trace amounts. The acceptable contents of the particular amino acids or sugar can be defined based on the characteristic in that cell culture is performed under such conditions that undifferentiated SCs, non-CMs and established cells are induced to undergo cell death without proliferating. The cell culture medium used in this invention is the one characterized in that the contents of the particular amino acids or sugar are less than 10%, preferably less than 5%, more preferably less than 1%, of those contents in a commercially available culture medium commonly used in cell culture. To cite some examples,
- Dulbecco's modified eagle's medium (DMEM, Sigma-Aldrich) is a culture medium containing 0.042 g/L of L-serine, 0.03 g/L of glycine, 0.584 g/L of L-glutamine, 0.84 g/L or 0.084 g/L of L-arginine HCl, and 4.5-10 g/L of sugar (D-glucose);
- F-12 culture medium (Sigma-Aldrich) is a culture medium containing 0.02102 or 0.0105 g/L of L-serine, 0.015014 g/L or 0.00751 g/L of glycine, 0.1460-2922 g/L of L-glutamine, 0.4214 or 0.211 g/L of L-arginine HCl, and 1.26-1.802 g/L of sugar (D-glucose); and
- RPMI 1640 culture medium (Sigma-Aldrich) is a culture medium containing 0.03-0.3 g/L of L-Serine, 0.01-0.1 g/L of glycine, 0.3 g/L of L-glutamine, 0.2-2 g/L of L-arginine, and 2.0-20.0 g/L of sugar (D-glucose). Therefore, when a culture medium is defined as being "free" of any of the particular amino acids and/or sugar, it is required that by reference to the constitution of a commercial culture medium commonly used in cell culture as mentioned above, the contents of those components be reduced to less than 10%, preferably less than 5%, more preferably less than 1%, of those contents in the commercial medium. Additionally, "sugar" as referred to herein is a concept that includes all sugars (*i.e.,* polysaccharides and monosaccharides (*e.g.*, glucose, galactose, fructose, mannose)) as found in a culture medium.

According to the first aspect as described above, undifferentiated SCs can be induced to undergo cell death by performing cell culture in a cell culture medium which is free of glutamine in the amino acid profile, or in a cell culture medium which is free of glutamine, as well as serine and glycine, in the amino acid profile, or in a cell culture medium which is free of glutamine, serine and glycine, as well as arginine, in the amino acid profile; and as a consequence, the present inventors can disclose a method for inducing cell death of undifferentiated SCs.

In the first aspect , in order to induce cell death of undifferentiated SCs, cell culture is continued under any of the aforementioned conditions free of the particular amino acids for 12-360 hours, preferably 24-240 hours, more preferably 48-120 hours.

As mentioned above, when the different culture conditions for CMs as defined in Patent Literature 2 are applied to human cells, surviving non-CMs or undifferentiated SCs may be present in small numbers depending on the condition. Accordingly, the present inventors then investigated whether CMs can be picked up more efficiently by combining the findings of the culture conditions for selectively obtaining CMs as disclosed in Patent Literature 2 with the findings of the culture conditions for inducing cell death of undifferentiated SCs as found in the first aspect of the present invention.

As a result, it was found that also in the case of human cells, CMs can be picked up more efficiently by selecting, from among the different culture conditions defined in Patent Literature 2, a low-glucose condition and a condition supplemented with lactate, pyruvate or a fatty acid which can be used as an energy source for CMs, and by combining these selected conditions with the culture conditions for inducing cell death of undifferentiated SCs as disclosed above in the present description.

On the basis of these results, the present inventors found that , by performing cell culture in culture media that are supplemented with a fatty acid, lactate or pyruvate, free of sugar, and free of glutamine in the amino acid profile (Lactate⁺, Gluc⁻, Gln⁻; or Pyr⁺, Gluc⁻, Gln⁻), undifferentiated SCs, such as PSCs including hESCs and hiPSCs, and other differentiated cells than CMs, can be induced to undergo cell death (FIGs. 2B, 3B and 4B), and consequently, CMs can be selectively picked up; thus, the inventors have completed this invention.

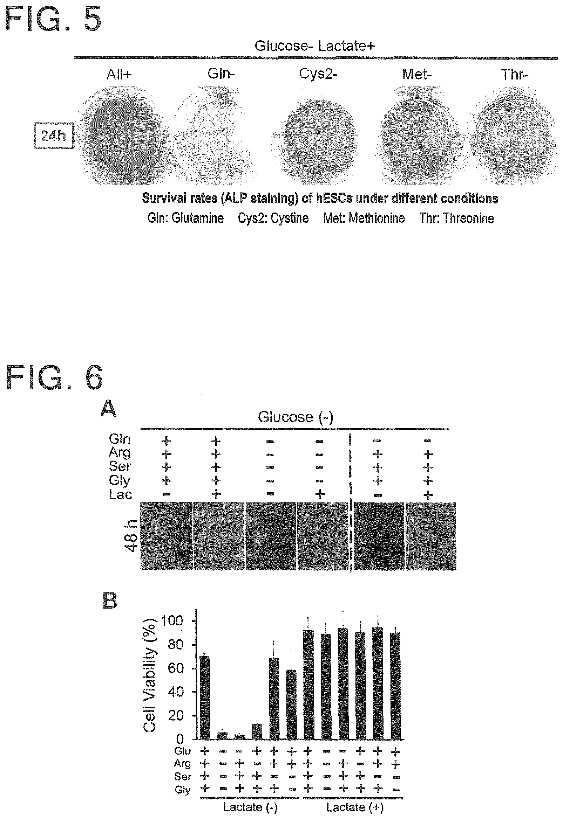

Further, in consideration of the fact that the consumptions of Cys2 and Met are relatively high, the present inventors investigated whether undifferentiated SCs (hESCs) are killed using culture media that are supplemented with lactate and free of sugar (Lactate⁺, Gluc⁻) and which are further free of either of the aforementioned amino acids. As a result, it was found that as compared to a lactate-supplemented, glucose- and glutamine-free medium (Lactate⁺, Gluc⁻, Gln⁻), a lactate-supplemented, glucose- and Cys2-free medium (Lactate⁺, Glue⁻, Cys2⁻), and a lactate-supplemented, glucose- and Met-free medium (Lactate⁺, Gluc⁻, Met⁻) have only comparable cell death-inducing activity to a lactate-supplemented, glucose- and threonine-free medium (Lactate⁺, Gluc⁻, Thr⁻) used as a control -- the cell death-inducing acvivity of the lactate-supplemented, glucose- and glutamine-free medium (Lactate⁺, Gluc⁻, Gln⁻) is most remarkable (FIG. 5). These results suggest that the glucose- and glutamine-free medium is superior in the ability to eliminate residual undifferentiated SCs over the threonine- or methionine-free media disclosed in Non-Patent Literatures 3 and 4.

Accordingly, in the second aspect , there is disclosed a cell culture medium characterized by being supplemented with lactate, pyruvate or a fatty acid (Lactate⁺ or Pyr⁺), free of glucose (Gluc⁻), and free of glutamine in the amino acid profile (Gln⁻). The cell culture medium of the second aspect of this invention may be further characterized by being free of serine and/or glycine (Ser⁻, Gly⁻) and still further characterized by being free of arginine (Arg⁻). Specific examples of the cell culture medium that can be used include, but are not limited to, the following:
- a cell culture medium that is supplemented with lactate, low in sugar, and free of glutamine in the amino acid profile (Lactate⁺, Glue⁻, Gln⁻);
- a cell culture medium that is supplemented with lactate, low in sugar, and free of glutamine and serine in the amino acid profile (Lactate⁺, Gluc⁻, Gln⁻, Ser⁻);
- a cell culture medium that is supplemented with lactate, low in sugar, and free of glutamine and glycine in the amino acid profile (Lactate⁺, Glue⁻, Gln⁻, Gly⁻);
- a cell culture medium that is supplemented with lactate, low in sugar, and free of glutamine, serine and glycine in the amino acid profile (Lactate⁺, Glue⁻, Gln⁻, Ser⁻, Gly⁻);
- a cell culture medium that is supplemented with lactate, low in sugar, and free of glutamine and arginine in the amino acid profile (Lactate⁺, Gluc⁻, Gln⁻, Arg");
- a cell culture medium that is supplemented with lactate, low in sugar, and free of glutamine, serine and arginine in the amino acid profile (Lactate⁺, Gluc⁻, Gln⁻, Ser⁻, Arg⁻);
- a cell culture medium that is supplemented with lactate, low in sugar, and free of glutamine, glycine and arginine in the amino acid profile (Lactate⁺, Gluc⁻, Gln⁻, Gly⁻, Arg⁻);
- a cell culture medium that is supplemented with lactate, low in sugar, and free of glutamine, serine, glycine and arginine in the amino acid profile (Lactate⁺, Glue⁻, Gln⁻, Ser⁻, Gly⁻, Arg⁻);
- a cell culture medium that is supplemented with pyruvate, low in sugar, and free of glutamine in the amino acid profile (Pyr⁺, Gluc⁻, Gln⁻);
- a cell culture medium that is supplemented with pyruvate, low in sugar, and free of glutamine and serine in the amino acid profile (Pyr⁺, Glue⁻, Gln⁻, Ser⁻);
- a cell culture medium that is supplemented with pyruvate, low in sugar, and free of glutamine and glycine in the amino acid profile (Pyr⁺, Gluc⁻, Gln⁻, Gly⁻);
- a cell culture medium that is supplemented with pyruvate, low in sugar, and free of glutamine, serine and glycine in the amino acid profile (Pyr⁺, Gluc⁻, Gln⁻, Ser⁻, Gly⁻);
- a cell culture medium that is supplemented with pyruvate, low in sugar, and free of glutamine and arginine in the amino acid profile (Pyr⁺, Gluc⁻, Gln⁻, Arg⁻);
- a cell culture medium that is supplemented with pyruvate, low in sugar, and free of glutamine, serine and arginine in the amino acid profile (Pyr⁺, Gluc⁻, Gln⁻, Ser⁻, Arg⁻;
- a cell culture medium that is supplemented with pyruvate, low in sugar, and free of glutamine, glycine and arginine in the amino acid profile (Pyr⁺, Gluc⁻, Gln⁻, Gly⁻, Arg⁻);
- a cell culture medium that is supplemented with pyruvate, low in sugar, and free of glutamine, serine, glycine and arginine in the amino acid profile (Pyr⁺, Gluc⁻, Gln⁻, Ser⁻, Gly⁻, Arg⁻);
- a cell culture medium that is supplemented with a fatty acid, low in sugar, and free of glutamine in the amino acid profile;
- a cell culture medium that is supplemented with a fatty acid, low in sugar, and free of glutamine and serine in the amino acid profile;
- a cell culture medium that is supplemented with a fatty acid, low in sugar, and free of glutamine and glycine in the amino acid profile;
- a cell culture medium that is supplemented with a fatty acid, low in sugar, and free of glutamine, serine and glycine in the amino acid profile;
- a cell culture medium that is supplemented with a fatty acid, low in sugar, and free of glutamine and arginine in the amino acid profile;
- a cell culture medium that is supplemented with a fatty acid, low in sugar, and free of glutamine, serine and arginine in the amino acid profile;
- a cell culture medium that is supplemented with a fatty acid, low in sugar, and free of glutamine, glycine and arginine in the amino acid profile;
- a cell culture medium that is supplemented with a fatty acid, low in sugar, and free of glutamine, serine, glycine and arginine in the amino acid profile.

The cell culture medium may be supplemented with ascorbate or albumin, or both. Supplementation with ascorbate and/or albumin does not affect the cell death-inducing activity of the cell culture medium.

The aforementioned cell culture medium of the second aspect is a culture medium free of serum or a serum alternative -- other conditions for the inventive medium, besides those of lactate, pyruvate and a fatty acid, glucose, and the particular amino acids (*i.e.,* glutamine, serine and/or glycine, or arginine), can be the same as those for common cell culture media (for example, Dulbecco's modified eagle's medium (DMEM), MEM culture media (e.g., α-MEM, MEM [Hank's BSS]), RPMI culture media (e.g., RPMI 1940), F12 culture medium, StemPro34, and mTeSR1).

According to the studies made by the present inventors, the aforementioned cell culture media having unique amino acid profiles are capable of inducing cell death of undifferentiated SCs, other differentiated cells than CMs, and established cells, but are not capable of inducing cell death of CMs. As a result, the cell culture medium of the second aspect can be used to select CMs from a mixture of CMs and non-CMs.

By stating that a cell culture medium is supplemented with lactate, it is meant that the cell culture medium to be used is supplemented with lactate to give a concentration of 0.1-10 mM. By stating that a cell culture medium is supplemented with pyruvate, it is meant that the cell culture medium to be used is supplemented with pyruvate (pyruvic acid) to give a concentration of 0.1-10 mM. By stating that a cell culture medium is supplemented with a fatty acid, it is meant that the cell culture medium to be used is supplemented with a medium-chain fatty acid (a fatty acid having 5-12 carbon atoms) or a long-chain fatty acid (a fatty acid having more than 12 carbon atoms). For example, oleate, linoleate, palmitate or the like can be added to give a concentration of 0.05-0.5 mM.

In contrast, as regards other components, when a cell culture medium is defined as being "free" of any of the particular amino acids (*i.e.*, serine, glycine, glutamine, and arginine) and/or sugar, it is ultimately desirable that the culture medium be completely free of those components, but to the extent that the object of the invention can be achieved, it is not necessarily required that the culture medium be deprived of 100% of those components.

According to the second aspect , non-CMs can be induced to undergo cell death by performing cell culture in a cell culture medium that is supplemented with lactate, pyruvate or a fatty acid, free of glucose, and free of glutamine in the amino acid profile, or in a lactate/pyruvate/fatty acid-supplemented, glucose-free cell culture medium which is free of glutamine, as well as serine and glycine, in the amino acid profile, or in a lactate/pyruvate/fatty acid-supplemented, glucose-free cell culture medium which is free of glutamine, serine and glycine, as well as arginine, in the amino acid profile; and as a consequence, the present inventors can disclose a method for inducing cell death of non-CMs and thereby selecting CMs by culturing a mixture of CMs and non-CMs. As referred to in this invention, "non-cardiomyocytes (non-CMs)" includes all types of cells other than CMs and cells that are to be induced in the future to differentiate into CMs -- for example, undifferentiated SCs, differentiated non-CMs, or established cells are included in "non-cardiomyocytes (non-CMs)".

In the second aspect , in order to induce cell death of non-CMs, cell culture is continued under any of the aforementioned conditions free of the particular amino acids for 12-360 hours, preferably 24-240 hours, more preferably 48-120 hours.

The cell culture medium may be supplemented with ascorbate or albumin, or both. Even when ascorbate and/or albumin is/are added, this addition does not have an influence on the cell death-inducing activity or CM purification accuracy of the cell culture medium, and CMs can be cultured for at least 600 hours.

The following presents working examples of the present invention. These examples are intended to describe the invention and not to limit the invention in any manner.

### EXAMPLES

### Example 1: Cell Culture

In this example, undifferentiated SCs (ESCs and iPSCs) were cultured, differentiated cells were cultured, and undifferentiated SCs were induced to differentiate into CMs.

The human ESCs (hESCs) were procured from Professor Norio Nakatsuji, Stem Cell Research Center, Institute for Frontier Medical Sciences, National Universtiy Corporation Kyoto University. The human iPSCs were procured from Professor Shinya Yamanaka, Center for iPS Cell Research and Application, National University Corporation Kyoto University. The hESCs and hiPSCs were subjected to undifferentiated state-maintaining culture using Matrigel (BD Bioscience, Cat No. 354277). The culture medium used was mTeSR1 (STEMCELL Technologies Inc., Cat No. 11875-119). Besides mTeSR1, any other culture medium, like Essential 8 (Life Technologies) or TeSR2 (STEMCELL Technologies Inc.), which is commonly used as a feeder-free medium, can also be used as a culture medium for maintaining undifferentiated state. Also, besides Matrigel, any other matrix, like Vitronectin (Life Technologies) or iMatrix-511 (Takara No. 892001), which is commonly used as a feeder-free matrix, can be used.

For passaging, hESC and hiPSC colonies were dissociated with CTK solution (Repro CELL) at 37°C for 5 minutes. Besides CTK solution, StemPro Accutase (Life Technologies No. 1110501) or TrypLE Express/Select (Life Technologies) can also be used for cell dissociation treatment.

In the present test, CM differentiation from undifferentiated SCs was induced using the following procedure.
- For CM differentiation, once the hESCs or hiPSCs reached 50-90% confluence, the medium was replaced with RPMI medium (Invitrogen) supplemented with B27 (without insulin; Invitrogen) and 6 µM CHIR99021 (Selleckchem or Wako) (day 0).
- On days 1-2, cell culture was performed in RPMI/B27-insulin(⁻) medium.
- On days 3-5, cell culture was performed in RPMI/B27-insulin(⁻) medium further supplemented with 5 µM IWP2 or 5 µM IWR-1 (Sigma 10161).
- On days 6-7, cell culture was performed in RPMI/B27-insulin(⁻) medium.
- On and after day 8, cell culture was performed in RPMI/B27-insulin(⁺) medium (Lian, X., et al., Nat Protocol, 2013, 8, 162-175). During days 8-11, beating CMs were observed to be present.

To establish the condition that the culture medium is free of as much sugar as possible, washing was done with PBS twice (Gluc⁻). Thereafter, cell culture was performed for 3-4 days in D-MEM culture medium supplemented with 4 mM lactate (Wako Pure Chemical, Cat No. 129-02666) (Lactate⁺), either in the presence of glutamine (Gln⁺) or in the absence of glutamine (Gln⁻). The lactate concentration needs to be adjusted to be in the range of 1-10 mM -- in this example, 4 mM lactate was added.

### Example 2: Amino Acid Requirements of Cultured Undifferentiated SCs under Culture Conditions

In this example, hESCs or hiPSCs were used as undifferentiated SCs and investigated to see what amino acids are required by the undifferentiated SCs under culture conditions.

In order to investigate nonessential and semi-essential amino acids particularly actively consumed in hESCs, amino acid concentrations in culture medium were measured. To be specific, for each type of cells, 1.5×10⁶ cells were cultured in a 3.5 cm dish, and then culture medium constitution was analyzed before and after cell culture.

Amino acid analysis was performed following the system of Shimbo, *et al.* (Shimbo, K., Rapid Commun. Mass Spectrom., 2009, 23, 1483-1492). After cell culture, the supernatants were each taken in a 1.5 mL tube and stored at -80°C until measurement. The samples were deproteinated, derivatized with an APDS reagent, and placed in the analyzer. Amino acid concentrations in the samples were determined using a calibration curve. The 37 amino acids analyzed are as follows: glycine (Gly), sarcosine (Sar), alanine (Ala), γ-aminobutyric acid (GABA), β-isoaminobutyric acid (b-AiBA), α-aminobutyric acid (a-ABA), serine (Ser), proline (Pro), valine (Val), threonine (Thr), taurine (Tau), hydroxyproline (HyPro), isoleucine (Ile), leucine (Leu), asparagine (Asn), ornithine (Orn), aspartate (Asp), glutamine (Gln), lysine (Lys), glutamate (Glu), methionine (Met), histidine (His), α-aminoadipic acid (a-AAA), hydroxylysine (HyLys), phenylalanine (Phe), 1-methylhistidine (1MeHis), 3-methylhistidine (3MeHis), arginine (Arg), citrulline (Cit), tyrosine (Tyr), tryptophan (Trp), cystathionine (Cysthi), carnosine (Car), anserine (Ans), cystine (Cys2), alanine-glutamine (Ala-Gln), and glycine-glutamine (Gly-Gln).

It was found that the concentrations of serine, arginine, cystine and glutamine in the medium particularly significantly decreased with the culture time.

Next, in order to determine the viability of cells cultured under different conditions, hESCs (FIG. 2) and hiPSCs (FIG. 4) were each investigated for their reactivity to different amino acid conditions by determining the alkaline phosphatase (ALP) activity of these cells, with those cells colored with StemTAG™ Alkaline Phosphatase Staining Kit (Sigma 86-R) being regarded as viable cells.

On the basis of the constitution of high-glucose DMEM (Invitrogen) ("Gluc⁺" in FIGs. 2A and 4A), there were prepared a culture medium free of all of the four amino acids (*i.e.,* glutamine, serine, glycine and arginine), as well as culture media each free of any one of these amino acids (*i.e*., serine alone, serine/glycine alone, arginine alone, and glutamine alone) in the basic constitution of said DMEM ("Ser⁻" "Ser⁻Gly⁻", "Arg⁻", and "Gln⁻", respectively). As a result of culturing hESCs and hiPSCs under each of the aforementioned culture conditions for about 48 hours and then subjecting the cultured cells to ALP staining, it was found that the number of hESC or hiPSC colonies was decreased by performing cell culture, though in the presence of glucose, under the serine-free condition (Ser⁻), the serine/glycine-free condition (Ser⁻Gly⁻), the glutamine-free condition (Gln⁻), or the arginine-free condition (Arg⁻). However, even after cell culture for 48 hours, ALP-positive undifferentiated cell colonies were observed in large numbers (FIGs. 2A, 4A).

Next, on the basis of the constitution of glucose-free DMEM (Invitrogen), with lactate (4 mM) being added thereto ("Gluc⁻, Lactate⁺" in FIGs. 2B and 4B), there were prepared a culture medium free of all of the four amino acids (*i.e.*, serine, glycine, arginine and glutamine) in the basis constitution of DMEM, as well as culture media each free of any one of these amino acids (*i.e*., serine alone, serine/glycine alone, arginine alone, and glutamine alone) in the basis constitution of DMEM ("Ser⁻", "Ser⁻Gly⁻", "Arg⁻" and "Gln⁻", respectively). As a result of culturing hESCs and hiPSCs under each of the aforementioned culture conditions for about 48 hours and then subjecting the cultured cells to ALP staining, it was found that in the sample groups cultured in the medium free of the four amino acids in the constitution of glucose-free DMEM, or in the medium free of glutamine alone (Gln⁻) in the constitution of glucose-free DMEM, ALP-positive hESCs or hiPSC colonies largely disappeared in 24 hours and completely disappeared in 48 hours (FIGs. 2B, 4B).

In consideration of FIG. 1 showing that the consumptions of Cys2 and Met in hESCs are relatively high, it was investigated whether undifferentiated SCs (hESCs) can survive even when cultured using culture media that are supplemented with lactate and free of sugar (Lactate⁺, Gluc⁻) and which are further free of either of the aforementioned amino acids. As for the culture media, on the basis of the constitution of glucose-free DMEM (Invitrogen), with lactate (4 mM) being added thereto ("Gluc⁻, Lactate⁺"), there were prepared and used culture media each free of any one of the four amino acids (*i.e*., glutamine alone, cystine alone, methionine alone, and threonine alone) in the basic constitution of said DMEM ("Gln⁻", "Cys2⁻", "Met⁻" and "Thr⁻", respectively). After cultured for 24 hours using each of the culture media, the cells were determined for their alkaline phosphatase (ALP) activity by coloring them with StemTAG™ Alkaline Phosphatase Staining Kit (Sigma 86-R) and observing those cells stained red as viable cells. As a result, it was found that as compared to a lactate-supplemented, glucose- and glutamine-free medium (Lactate⁺, Gluc⁻, Gln⁻), a lactate-supplemented, glucose- and Cys2-free medium (Lactate⁺, Gluc⁻, Cys2⁻), and a lactate-supplemented, glucose- and Met-free medium (Lactate⁺, Gluc⁻, Met⁻) have only comparable cell death-inducing activity to a lactate-supplemented, glucose- and threonine-free medium (Gluc⁻, Lactate⁺, Thr⁻) used as a control -- the cell death-inducing acvivity of the lactate-supplemented, glucose- and glutamine-free medium (Gluc⁻, Lactate⁺, Gln⁻) is most remarkable (FIG. 5). These results suggest that the glucose- and glutamine-free medium is superior in the ability to eliminate residual undifferentiated SCs over the threonine- or methionine-free media disclosed in Non-Patent Literatures 3 and 4.

### Example 3: Cell Reactivity to Different Culture Medium Conditions

In this example, it was clarified how the survival rates of undifferentiated SCs and CMs change when culturing them under different culture medium conditions.

As for culture media used in this example, on the basis of the constitution of glucose-free DMEM (Invitrogen), either with lactate (4 mM) being added thereto (Gluc⁻, Lactate⁺) or without lactate being added thereto (Gluc⁻, Lactate⁻), there were prepared culture media each further free of any one of the four amino acids (*i.e.*, arginine alone, glutamine alone, serine alone, and glycine alone) ("Arg"", "Gln⁻", "Ser⁻" and "Gly⁻", respectively). After undifferentiated SCs (hESCs) or murine neonatal CMs were cultured for about 24 hours under each of the aforementioned culture conditions, the cells were stained using a live/dead cell double staining kit (TaKaRa Bio, Inc.), imaged (FIGs. 3A and 6A), and counted to determine their cell survival rates (FIGs. 3B and 6B).

FIG. 3 shows the results of observing the survival or death of the undifferentiated SCs (hESCs) cultured under the different conditions free of any one of the different nonessential amino acids which were highly consumed as per FIG. 1, and on that basis, determining their cell survival rates. The results revealed that the survival rate of undifferentiated SCs was significantly decreased by culturing them in the glutamine-free culture media regardless of being supplemented or not supplemented with lactate ("Lactate⁺, Gluc⁻, Gln⁻" or "Lactate", Gluc⁻, Gln⁻") (FIG. 3).

FIG. 6 shows the results obtained in the same way by observing the survival or death of the murine neonatal CMs cultured under the different conditions free of any one of the different nonessential amino acids which were highly consumed as per FIG. 1, and on that basis, by determining their cell survival rates. The results revealed that the survival rate of the neonatal CMs was significantly improved by culturing them in the culture medium free of glutamine but supplemented with lactate (Lactate⁺, Gluc⁻, Gln⁻), or in other words that the cell death-inducing activity of a glutamine-free medium (Gln⁻) is reduced by adding lactate (FIG. 6).

### Example 4: Induction of Cardiomyocyte (CM) Differentiation from iPSCs under Culture Conditions

This example was intended to clarify the procedure for inducing CM differentiation from hiPSCs.
- For CM differentiation, once hiESCs reached 50-90% confluence, the medium was replaced with RPMI medium (Invitrogen) supplemented with B27 (without insulin; Invitrogen) and 6 µM CHIR99021 (Selleckchem or Wako) (day 0).
- On days 1-2, cell culture was performed in RPMI/B27-insulin(⁻) medium.
- On days 3-5, cell culture was performed in RPMI/B27-insulin(⁻) medium further supplemented with 5 µM IWP2 or 5 µM IWR-1.
- On days 6-7, cell culture was performed in RPMI/B27-insulin(⁻) medium.
- On and after day 8, cell culture was performed in RPMI/B27-insulin(⁺) medium (Lian, X., et al., Nat Protocol, 2013, 8, 162-175). During days 8-11, beating CMs were observed to be present (FIG. 4A).

To establish the condition that the culture medium is substantially free of sugar, washing was done with PBS twice (Gluc⁻). Thereafter, cell culture was performed for 3-4 days in D-MEM culture medium supplemented with 4 mM lactate (Wako Pure Chemical, Cat No. 129-02666) (Lactate⁺), either in the presence of glutamine (Gln⁺) or in the absence of glutamine (Gln⁻). The lactate concentration needs to be adjusted to be in the range of 1-10 mM -- in this example, 4 mM lactate was added.

### Example 5: Amino Acid Requirements of Differentiated CMs under Culture Conditions

In this example, the CMs into which hiPSCs were induced to differentiate were investigated to see what amino acids are required by the differentiated CMs under culture conditions.

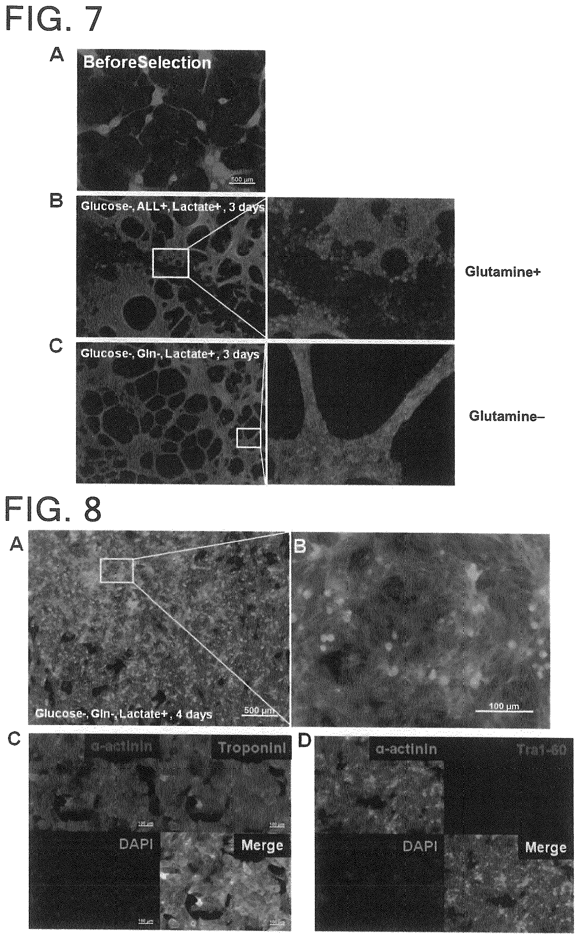

FIG. 7 shows the photos of the cells that were cultured for 3 days in a glucose-supplemented medium (FIG. 7A), a glucose-free, lactate-supplemented medium (Cell Stem Cell, 2013 12:127-37) (Gluc⁻, All⁺, Lactate⁺) (FIG. 7B) or a lactate-supplemented, glucose- and glutamine-free medium (Gluc⁻, Gln⁻, Lactate⁺) (FIG. 7C), and then subjected to immunostaining.

For immunostaining, the cells were immobilized with 4% paraformaldehyde for 15-30 minutes, and then stained with DAPI (Invitrogen) for cell nuclei, and with Anti-α-Actinin Antibody (Sigma) and Alexa 546 Donkey Anti-Mouse IgG (Invitrogen) for striated structures, to thereby detect fluorescence emissions.

According to the immunostaining images captured after cell culture for 3 days, just a few of the cells cultured in the glucose-free, lactate-supplemented medium (with glutamine) (Gluc⁻, Gln⁺, Lactate⁺) and having nuclei stained with DAPI were observed to be negative for α-Actinin as a CM marker (FIG. 7B), whereas nearly 100% of the cells cultured in the lactate-supplemented, glucose- and glutamine-free medium (Gluc⁻, Gln⁻, Lactate⁺) and having nuclei stained with DAPI were positive for α-Actinin (FIG. 7C).

These results indicate that under the glutamine-supplemented culture condition (FIG. 7B), other types of cells than CMs survived in small numbers, whereas under the glutamine-free culture condition (FIG. 7C), CMs survived but other types of cells than CMs (*i.e.,* undifferentiated SCs, non-CMs) were induced to undergo cell death and completely disappeared.

According to the predication of the mechanism by which this difference may occur in connection with the sugar metabolic pathways, it was anticipated that in the case of common cells (undifferentiated SCs and non-CMs), the pyruvate-supplying pathway from the glycolytic system, and the α-ketoglutarate-supplying pathway from glutamine via glutamate, in the sequential flow of sugar metabolism, contribute greatly to cell survival, but the pyruvate-supplying pathway from lactate is generally maintained to contribute significantly less to cell survival. In contrast, it was anticipated that in the case of CMs, not only the pyruvate-supplying pathway from the glycolytic system and the α-ketoglutarate-supplying pathway from glutamine via glutamate, but also the pyruvate-supplying pathway from lactate, contribute substantially to CM survival.

As a result, it was found that CMs can be selectively picked up more efficiently and in a shorter time by culturing CMs induced from hESCs or hiPSCs (*i.e.,* CMs in admixture with undifferentiated SCs and non-CMs) under the lactate-supplemented, glucose- and glutamine-free culture condition (Gluc⁻, Gln⁻, Lactate⁺), as compared to the case of culturing the cells under the lactate-supplemented, glucose-free culture condition (with glutamine) (Gluc⁻, Gln⁺, Lactate⁺) (FIG. 7B).

FIG. 8 shows the CM masses that were cultured for 4 days in the lactate-supplemented, glucose- and glutamine-free medium (Gluc⁻, Gln⁻, Lactate⁺), dissociated with 0.25% Trypsin EDTA, seeded on a culture dish coated with fibronectin (Sigma), and subjected to immunostaining. For immunostaining, the cells were immobilized with 4% paraformaldehyde and then stained with Anti-α-Actinin Antibody (Sigma), Anti-Troponin I Antibody (Santacruz), and Anti-Tra1-60 (Millipore). Here, α-Actinin and Troponin I are CM-specific markers, and Tra1-60 is a PSC-specific marker.

The results showed that almost all of the cells that were nuclear stained with DAPI were positive for α-Actinin and Troponin I (FIGs. 8A and 8B). To confirm whether the cultured cells were CMs, the cells were subjected to overlapped staining for α-Actinin and Troponin I, and as a result, α-Actinin-stained cells were completely in accord with Troponin I-stained cells (FIG. 8C). Also, to confirm the presence of residual undifferentiated SCs, the cultured cells were analyzed for the presence of Tra1-60-positive cells, and as a result, all of the cells were negative for Tra1-60 (upper right panel in FIG. 8D). Accordingly, undifferentiated SCs were confirmed to be completely eliminated.

### Example 6: Detection of Residual Undifferentiated SCs

As a detection method for residual undifferentiated SCs, there was reported a highly sensitive method for qualifying the expression of the Lin28 gene by Q-PCR (PLOS ONE, 2012; 7(5): e37342). In each of a lactate-supplemented, glucose-free medium (Gluc⁻, All⁺, Lactate⁺) and a lactate-supplemented, glucose- and glutamine-free medium (Gluc⁻, Gln⁻, Lactate⁺), hiPSCs were induced to differentiate into CMs, and each vial of cells was refined and purified for 4 days and subjected to mRNA extraction to prepare cDNA. As a result of normalizing expression levels with 18S, Lin28 was expressed in the cells cultured in the lactate-supplemented, glucose-free medium (Gluc⁻, All⁺, Lactate⁺), whereas this marker was not expressed at all in the cells cultured in the lactate-supplemented, glucose- and glutamine-free medium (Gluc⁻, Gln⁻, Lactate⁺) (FIG. 9). These results showed that the lactate-supplemented, glucose- and glutamine-free medium (Gluc⁻, Gln⁻, Lactate⁺) is superior in the ability to eliminate undifferentiated SCs over the lactate-supplemented, glucose-free medium (Gluc⁻, All⁺, Lactate⁺).

### Example 7: Lactate Fluxome (Metabolic Flux) Analysis in CMs under Culture in Glucose- and Glutamine-free Medium

In this example, the mechanism of how CMs metabolize lactate and survive under a glucose- and glutamine-free culture condition was investigated using capillary electrophoresis and mass spectrometry.

For each of hESCs and murine neonatal CMs, the culture medium was switched to modified glucose-free DMEM (Invitrogen) supplemented with 4 mM [¹³C]-labeled lactate (Isotec). After one hour, the cells were washed in 10% mannitol (Wako) and plunged into methanol containing internal standards (200 µM L-methionine sulfone for cations, and 200 µM MES for anions). After the cells and the culture media were collected, capillary electrophoresis and mass spectrometry were conducted using an Agilent capillary electrophoresis system equipped with an air pressure pump, an Agilent 1100 series mass selective detector mass spectrometer, an Agilent 1100 series isocratic high-performance liquid-chromatography pump, a G1603A Agilent capillary electrophoresis and mass spectrometry adaptor kit, and a G1607A Agilent capillary electrophoresis and mass spectrometry sprayer kit (Agilent Technologies). Values were corrected against cell numbers. In CMs, metabolites of the TCA cycle were detected in significantly large amounts (FIGs. 10A, 10B). However, in hESCs, the metabolites were observed only in small amounts (FIG. 10B). This suggested that in CMs, as compared to non-CMs including ESCs, exogenous lactate is more efficiently metabolized through the TCA cycle. Surprisingly, [¹³C] labels were detected in significantly larger amounts in 2-oxoglutarate and glutamate in CMs (FIGs. 10C, 10D). These results not only indicate that lactate greatly contributes to ATP production through the TCA cycle, but also suggest that even under culture in a glutamine- and glutamate-free medium, lactate constributes to the biosynthesis of 2-oxoglutarate and glutamate and compensates for them, and this explains why CMs can survive.

### Example 8: Effect of Culturing Cell Group Obtained by Inducing Differentiation of hiPSCs, under Lactate-Supplemented, Glucose- and Glutamine-free Culture Condition

Following the same procedure as used in Example 4, hiPSCs were induced to differentiate into CMs. For refinement and purification of the obtained cell group (hiPSC-CMs), there was prepared a lactate-supplemented, glucose- and glutamine-free medium (Gluc⁻, Gln⁻, Lac⁺) on the basis of the constitution of DMEM (Invitrogen), and the cell group was cultured in the prepared medium for 5 days.

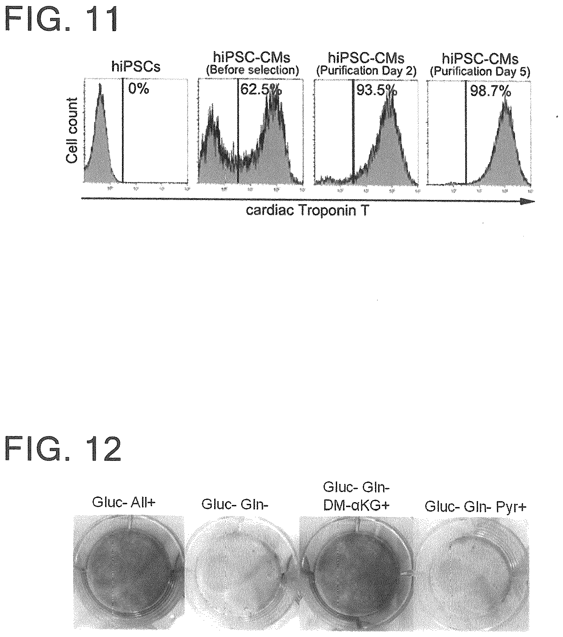

On days 2 and 5 from the start of culture under the lactate-supplemented, glucose- and glutamine-free condition (Gluc⁻, Gln⁻, Lac⁺), the cell group was analyzed by FACS. The results are shown in FIG. 11. According to the FACS data, the percentage of Troponin T-positive cells was 93.5% in the cell group on day 2 and 98.7% in the cell group on day 5.

This revealed that CMs can be purified by performing cell culture under the glucose- and glutamine-free condition supplemented with lactate as an energy source for CMs (Gluc⁻, Gln⁻, Lac⁺).

### Example 9: Viability of Undifferentiated SCs and CMs under Pyruvate-supplemented, Glutamine-free Culture Condition

In this example, it was investigated whether hESCs used as undifferentiated SCs, and murine neonatal CMs used as CMs, can survive when cultured under a pyruvate-supplemented, glutamine-free culture condition.

On the basis of the constitution of glucose-free DMEM (Invitrogen), there were prepared a medium containing all amino acids in the basis constitution of DMEM ("Gluc⁻, ALL⁺" condition), a medium free of glutamine in the basis constitution of DMEM ("Gluc⁻", Gln⁻" condition), a medium free of glutamine in the basis constitution of DMEM but supplemented with 4 mM α-ketoglutarate ("Gluc⁻, Gln⁻, DM-αKG⁺"), and a medium free of glutamine in the basis constitution of DMEM but supplemented with 2 mM pyruvate ("Gluc⁻, Gln⁻, Pyr⁺"). In each of the aforementioned culture media, hESCs were cultured for 48 hours. Then, the hESCs were determined for their alkaline phosphatase (ALP) activity by coloring them with StemTAG™ Alkaline Phosphatase Staining Kit (Sigma 86-R) and observing those cells stained red as viable cells (FIG. 12). As a result, ALP-positive undifferentiated cell colonies were observed in large numbers in the α-ketoglutarate-supplemented medium, whereas no ALP-positive undifferentiated cell colony was present in the pyruvate-supplemented medium. This revealed that under a glutamate-free condition, undifferentiated cells undergo cell death in a short time even in the presence of pyruvate.

Next, for the purpose of use in murine neonatal CMs, there were prepared, on the basis of the constitution of glucose-free DMEM (Invitrogen), a medium containing all amino acids in the basis constitution of DMEM (Gluc⁻, ALL⁺), a medium free of glutamine in the basis constitution of DMEM (Gluc⁻, Gln⁻), a medium free of glutamine in the basis constitution of DMEM but supplemented with 2 mM pyruvate (Gluc⁻, Gln⁻, Pyr⁺), and a medium free of glutamine in the basis constitution of DMEM but supplemented with 4 mM lactate (Gluc⁻, Gln⁻, Lac⁺). Murine neonatal CMs were cultured for 48 hours in each of the aforementioned culture media, stained with a live/dead cell double staining kit (TaKaRa Bio, Inc.), and imaged (FIG. 13). Green-stained cells represent viable cells, while red-stained cells represent apoptotic cells. Under a glucose- and glutamine-free culture condition, the survival rate of the CMs cultured in the presence of lactate or pyruvate as an energy source for CMs was significantly higher than that of the CMs cultured in the absence of lactate and pyruvate. This revealed that the cell death-inducing activity of a glutamine-free medium (Gln⁻) on CMs is reduced by adding pyruvate.

### Example 10: Detection of Residual Undifferentiated SCs

This example investigated the detection sensitivity of another detection method for residual undifferentiated SCs using a combination of laminin-521 and Essential 8 medium (Tano, et al., PLOS ONE, 2014). The method using mTeSR1 as a culture medium and iMatrix (produced by Nippi, Inc.) as a scaffolding material was evaluated for its sensitivity in detecting undifferentiated SCs. At first, HEK293 cell groups each mixed with no hiPSCs (0%) or with 0.1%, 0.01% or 0.001% hiPSCs were imaged (FIG. 14A). Since hiPSCs were detected even in the cell group mixed with 0.001% hiPSCs, it was confirmed that this detection method is capable of detecting undifferentiated SCs present in a proportion as low as 0.001%.

Next, differentiated cells from hiPSCs were evaluated for the presence of residual undifferentiated SCs. First, following the same procedure as used in Example 4, hiPSCs were induced to differentiate into CMs, and the cells were imaged (FIG. 14B). Then, for refinement and purification of cell groups induced to differentiate under the same conditions, there were prepared a lactate-supplemented, glucose-free and glutamine-supplemented medium (Gluc⁻, Gln⁺, Lac⁺) based on the constitution of DMEM (Invitrogen), and a lactate-supplemented, glucose- and glutamine-free medium (Gluc⁻, Gln⁻, Lac⁺) based on the constitution of DMEM (Invitrogen), and the cell groups were cultured in each of these culture media for 4 days and then imaged (FIG. 14B). TRA1-60-positive cells were observed in the cell group before refinement and purification, and in the cell group refined and purified under the 4mM lactate-supplemented, glucose-free, and 4 mM glutamine-supplemented condition (Gluc⁻, Gln⁺, Lac⁺). In contrast, no TRA1-60-positive cells were detected in the cell group refined and purified under the lactate-supplemented, glucose- and glutamine-free condition (Gluc⁻, Gln⁻, Lac⁺).

This revealed that the survival rate of residual undifferentiated SCs is less than 0.001% under the lactate-supplemented, glucose- and glutamine-free culture condition (Gluc⁻, Gln⁻, Lac⁺).

### Example 11: Cell death-Inducing Activity on Proliferative Non-CMs

This example investigated the cell death-inducing activity of a glutamine-free culture medium on proliferative non-CMs.

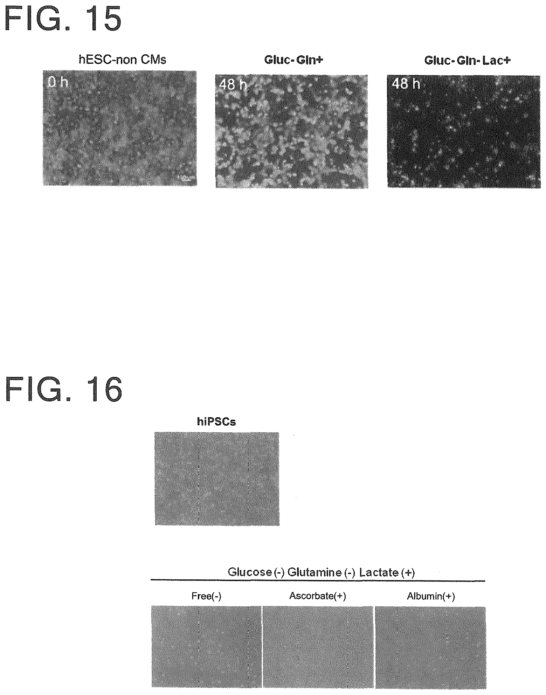

First, hESCs were cultured under the condition of DMEM (with 10% FBS, without bFGF) for 2 weeks to obtain a proliferative non-CM group not including CMs. This cell group includes fibroblasts and other differentiated cells than CMs. After the cell group was cultured for 48 hours under a glucose-free, 4 mM glutamine-supplemented culture condition (Gluc⁻, Gln⁺) based on the constitution of DMEM (Invitrogen), viable cells were observed to be present (FIG. 15). In contrast, after proliferative non-CMs were cultured for 48 hours under a 4 mM lactate-supplemented, glucose- and glutamine-free condition (Gluc⁻, Gln⁻, Lac⁺), no viable cells were observed to be present at all and the cultured cells underwent complete cell death (FIG. 15).

This revealed that proliferative non-CMs can be induced to undergo cell death under the lactate-supplemented, glucose- and glutamine-free condition. In recent years, it was reported that tumor formation after cell transplantation is caused not only by undifferentiated SCs but also by immature proliferative cells (Nori, et al., Stem Cell Report, 2015). However, this inventive method can induce cell death of not only undifferentiated SCs but also proliferative cells in a short time.

### Example 12: Compounds That Can be Added to Purification Media

This example searched for a condition under which undifferentiated SCs and differentiated non-CMs do not survive but only CMs can survive for a longer time when cell culture was performed in a purification culture medium supplemented with a compound that is nutritional for CMs.

On the basis of the constitution of DMEM (Invitrogen), there were prepared lactate-supplemented, glucose- and glutamine-free culture media (Gluc⁻, Gln⁻, Lac⁺) which are each further supplemented with ascorbate (25 mg/L) or albumin (0.1%).

Next, hiPSCs were cultured in each of the prepared media. After 24 hours from the start of culture, the hiPSCs cultured in both of the media were observed to undergo cell death (FIG. 16).

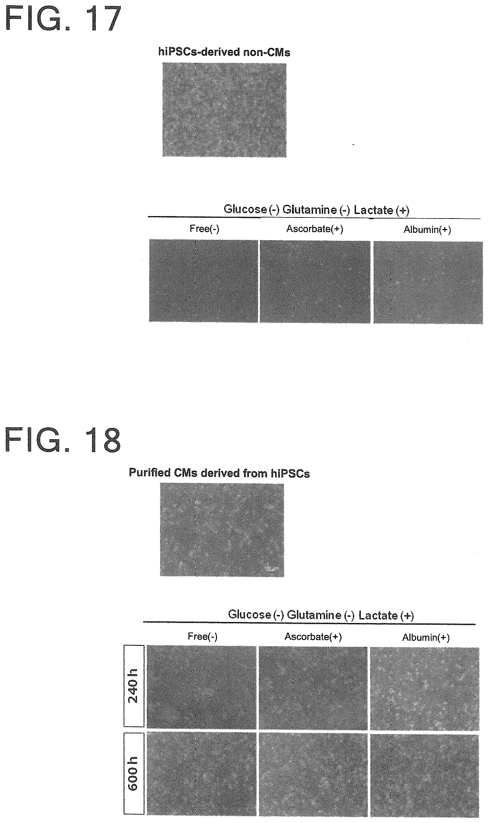

Next, hiPSCs were cultured under the condition of DMEM (with 10% FBS, without bFGF) for 2 weeks to obtain a proliferative non-CM group not including CMs. The obtained cell group was cultured in each of the prepared media. After 48 hours from the start of culture, the cells were observed to undergo cell death (FIG. 17).

Next, following the same procedure as used in Example 4, hiPSCs were induced to differentiate into CMs. After the obtained cell masses were cultured for 600 hours in each of the media supplemented with ascorbate or albumin, CMs were observed to survive (FIG. 18).

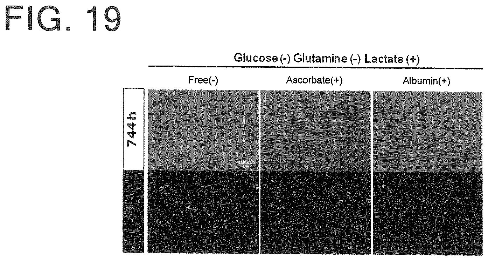

Further, cell culture was continued until 744 hours, and as a result, beating CMs were still observed to be present in large numbers under both of the culture conditions. Next, cell staining was performed with the dead cell staining fluorescent dye PI (propidium iodide) and the stained cells were observed (FIG. 19). Live cells were observed in larger amounts under the ascorbate- or albumin-supplemented condition than under the ascorbate- and albumin-free condition ("Free(⁻)"). The above results revealed that CMs can be purified using each of the glucose- and glutamine-free culture media which are supplemented with lactate as an energy source for CMs and further supplemented with ascorbate or albumin. The results also showed that CMs can be allowed to survive for a longer time by using these inventive culture media.

### INDUSTRIAL APPLICABILITY

According to the first aspect, there is disclosed a cell culture medium for use in inducing cell death of undifferentiated SCs by preparing a cell culture medium that is free of glutamine in the amino acid profile. Simply by performing cell culture using the cell culture medium of the first aspect, undifferentiated SCs can be easily induced to undergo cell death. Also, according to the second aspect , there is disclosed a cell culture medium for use in selecting CMs by preparing a cell culture medium that is supplemented with lactate, pyruvate or a fatty acid, free of sugar (glucose), and free of glutamine in the amino acid profile. Simply by performing cell culture using the cell culture medium of the second aspect , undifferentiated SCs, such as PSCs including hESCs and hiPSCs, as well as other differentiated cells than CMs, and established cells, as defined below, can be easily induced to undergo cell death, and consequently, CMs can be selected.

## Claims

1. Use of a cell culture medium which has less than 10% of glutamine in the amino acid profile for inducing cell death of undifferentiated stem cells.

2. A method for inducing cell death of undifferentiated stem cells by performing cell culture in a cell culture medium that has less than 10 % of glutamine in the amino acid profile.

3. The use according to claim 1 or the method according to claim 2, wherein the undifferentiated stem cells are pluripotent stem cells or multipotent stem cells.

4. The use according to claim 1 or 3 or the method according to claim 2 or 3, wherein the cell culture medium is further free of serine and glycine in the amino acid profile.

5. The use according to any one of claims 1, 3 and 4 and 5 or the method according to any one of claims 2-4, wherein the cell culture medium is further free of arginine in the amino acid profile.

6. The method according to any one of claims 2-5, wherein the cell culture is continued for 12-360 hours under any of the above particular amino acid-free conditions.

7. Use of a cell culture medium which is:
supplemented with lactate, free of sugar, and has less than 10 % of glutamine in the amino acid profile; or
supplemented with pyruvate, free of sugar, and has less than 10 % of glutamine in the amino acid profile; or
supplemented with a fatty acid, free of sugar, and has less than 10 % of glutamine in the amino acid profile,
for selecting cardiomyocytes from a mixture of cardiomyocytes and non-cardiomyocytes.

8. The use according to claim 7, wherein the non-cardiomyocytes are cells including undifferentiated stem cells, other differentiated cells than cardiomyocytes, and established cells.

9. A method for selecting cardiomyocytes, comprising:
culturing a mixture of cardiomyocytes and non-cardiomyocytes in a cell culture medium which is supplemented with lactate, free of sugar, and has less than 10 % of glutamine in the amino acid profile; or
culturing a mixture of cardiomyocytes and non-cardiomyocytes in a cell culture medium which is supplemented with pyruvate, free of sugar, and has less than 10 % of glutamine in the amino acid profile; or
culturing a mixture of cardiomyocytes and non-cardiomyocytes in a cell culture medium which is supplemented with a fatty acid, free of sugar, and and has less than 10 % of glutamine in the amino acid profile.

10. The method according to claim 9, wherein the non-cardiomyocytes are undifferentiated stem cells, differentiated non-cardiomyocytes, or established cells.

11. The use according to claim 7 or the method according to claim 9 or 10, wherein the cell culture medium is further free of serine and glycine in the amino acid profile.

12. The use according to claim 7 or 11 or the method according to any one of claims 9-11, wherein the cell culture medium is further free of arginine in the amino acid profile.

13. The method according to any one of claims 9-12, wherein the cell culture is continued for 12-360 hours under any of the above particular amino acid-free conditions.

14. The use according to any one of claims 7, 8, 11 and 12 or the method according to any one of claims 9-13, wherein the cell culture medium is supplemented with ascorbate or albumin.

## Patentansprüche

1. Verwendung eines Zellkulturmediums, das weniger als 10% Glutamin in dem Aminosäureprofil aufweist, zum Induzieren von Zelltod von undifferenzierten Stammzellen.

2. Verfahren zum Induzieren von Zelltod von undifferenzierten Stammzellen durch Durchführen einer Zellkultur in einem Zellkulturmedium, das weniger als 10% Glutamin in dem Aminosäureprofil aufweist.

3. Verwendung nach Anspruch 1 oder Verfahren nach Anspruch 2, wobei die undifferenzierten Stammzellen pluripotente Stammzellen oder multipotente Stammzellen sind.

4. Verwendung nach Anspruch 1 oder 3 oder Verfahren nach Anspruch 2 oder 3, wobei das Zellkulturmedium des Weiteren frei von Serin und Glycin in dem Aminosäureprofil ist.

5. Verwendung nach einem der Ansprüche 1, 3 und 4 und 5 oder Verfahren nach einem der Ansprüche 2 bis 4, wobei das Zellkulturmedium des Weiteren frei von Arginin in dem Aminosäureprofil ist.

6. Verfahren nach einem der Ansprüche 2 bis 5, wobei die Zellkultur für 12 bis 360 Stunden unter einer der obigen bestimmten Aminosäure-freien Bedingungen fortgesetzt wird.

7. Verwendung eines Zellkulturmediums, das:
mit Lactat supplementiert ist, frei von Zucker ist und weniger als 10% Glutamin in dem Aminosäureprofil aufweist; oder
mit Pyruvat supplementiert ist, frei von Zucker ist und weniger als 10% Glutamin in dem Aminosäureprofil aufweist; oder
mit einer Fettsäure supplementiert ist, frei von Zucker ist und weniger als 10% Glutamin in dem Aminosäureprofil aufweist,
zum Selektieren von Cardiomyocyten aus einem Gemisch aus Cardiomyocyten und Nicht-Cardiomyocyten.

8. Verwendung nach Anspruch 7, wobei die Nicht-Cardiomyocyten Zellen einschließlich undifferenzierter Stammzellen, anderer differenzierter Zellen als Cardiomyocyten und etablierter Zellen sind.

9. Verfahren zum Selektieren von Cardiomyocyten, umfassend:
Züchten eines Gemischs aus Cardiomyocyten und Nicht-Cardiomyocyten in einem Zellkulturmedium, das mit Lactat supplementiert ist, frei von Zucker ist und weniger als 10% Glutamin in dem Aminosäureprofil aufweist; oder
Züchten eines Gemischs aus Cardiomyocyten und Nicht-Cardiomyocyten in einem Zellkulturmedium, das mit Pyruvat supplementiert ist, frei von Zucker ist und weniger als 10% Glutamin in dem Aminosäureprofil aufweist; oder
Züchten eines Gemischs aus Cardiomyocyten und Nicht-Cardiomyocyten in einem Zellkulturmedium, das mit einer Fettsäure supplementiert ist, frei von Zucker ist und weniger als 10% Glutamin in dem Aminosäureprofil aufweist.

10. Verfahren nach Anspruch 9, wobei die Nicht-Cardiomyocyten undifferenzierte Stammzellen, differenzierte Nicht-Cardiomyocyten oder etablierte Zellen sind.

11. Verwendung nach Anspruch 7 oder Verfahren nach Anspruch 9 oder 10, wobei das Zellkulturmedium des Weiteren frei von Serin und Glycin in dem Aminosäureprofil ist.

12. Verwendung nach Anspruch 7 oder 11 oder Verfahren nach einem der Ansprüche 9 bis 11, wobei das Zellkulturmedium des Weiteren frei von Arginin in dem Aminosäureprofil ist.

13. Verfahren nach einem der Ansprüche 9 bis 12, wobei die Zellkultur für 12 bis 360 Stunden unter einer der obigen bestimmten Aminosäure-freien Bedingungen fortgesetzt wird.

14. Verwendung nach einem der Ansprüche 7, 8, 11 und 12 oder Verfahren nach einem der Ansprüche 9 bis 13, wobei das Zellkulturmedium mit Ascorbat oder Albumin supplementiert ist.

## Revendications

1. Utilisation d'un milieu de culture cellulaire dont le profil d'acides aminés comporte moins de 10 % de glutamine pour induire la mort cellulaire de cellules souches indifférenciées.

2. Procédé pour induire la mort cellulaire de cellules souches indifférenciées en réalisant une culture cellulaire dans un milieu de culture cellulaire dont le profil d'acides aminés comporte moins de 10 % de glutamine.

3. Utilisation selon la revendication 1 ou procédé selon la revendication 2, où les cellules souches indifférenciées sont des cellules souches pluripotentes ou des cellules souches multipotentes.

4. Utilisation selon la revendication 1 ou 3 ou procédé selon la revendication 2 ou 3, où le profil d'acides aminés du milieu de culture cellulaire est en outre dépourvu de sérine et de glycine.

5. Utilisation selon l'une quelconque des revendications 1, 3 et 4 et 5 ou procédé selon l'une quelconque des revendications 2 à 4, où le profil d'acides aminés du milieu de culture cellulaire est en outre dépourvu d'arginine.

6. Procédé selon l'une quelconque des revendications 2 à 5, dans lequel la culture cellulaire est poursuivie pendant 12-360 heures dans l'une quelconque des conditions dépourvues d'acides aminés particulières ci-dessus.

7. Utilisation d'un milieu de culture cellulaire qui est :
supplémenté avec du lactate, dépourvu de sucre, et dont le profil d'acides aminés comporte moins de 10 % de glutamine ; ou
supplémenté avec du pyruvate, dépourvu de sucre, et dont le profil d'acides aminés comporte moins de 10 % de glutamine ; ou
supplémenté avec un acide gras, dépourvu de sucre, et dont le profil d'acides aminés comporte moins de 10 % de glutamine,
pour sélectionner des cardiomyocytes à partir d'un mélange de cardiomyocytes et de non-cardiomyocytes.

8. Utilisation selon la revendication 7, dans laquelle les non-cardiomyocytes sont des cellules comprenant des cellules souches indifférenciées, des cellules différenciées autres que des cardiomyocytes, et des cellules établies.

9. Procédé pour sélectionner des cardiomyocytes, comprenant :
la mise en culture d'un mélange de cardiomyocytes et de non-cardiomyocytes dans un milieu de culture cellulaire qui est supplémenté avec du lactate, dépourvu de sucre, et dont le profil d'acides aminés comporte moins de 10 % de glutamine ; ou
la mise en culture d'un mélange de cardiomyocytes et de non-cardiomyocytes dans un milieu de culture cellulaire qui est supplémenté avec du pyruvate, dépourvu de sucre, et dont le profil d'acides aminés comporte moins de 10 % de glutamine ; ou
la mise en culture d'un mélange de cardiomyocytes et de non-cardiomyocytes dans un milieu de culture cellulaire qui est supplémenté avec un acide gras, dépourvu de sucre, et dont le profil d'acides aminés comporte moins de 10 % de glutamine.

10. Procédé selon la revendication 9, dans lequel les non-cardiomyocytes sont des cellules souches indifférenciées, des non-cardiomyocytes différenciés, ou des cellules établies.

11. Utilisation selon la revendication 7 ou procédé selon la revendication 9 ou 10, où le profil d'acides aminés du milieu de culture cellulaire est en outre dépourvu de sérine et de glycine.

12. Utilisation selon la revendication 7 ou 11 ou procédé selon l'une quelconque des revendications 9 à 11, où le profil d'acides aminés du milieu de culture cellulaire est en outre dépourvu d'arginine.

13. Procédé selon l'une quelconque des revendications 9 à 12, dans lequel la culture cellulaire est poursuivie pendant 12 à 360 heures dans l'une quelconque des conditions dépourvues d'acides aminés particulières ci-dessus.

14. Utilisation selon l'une quelconque des revendications 7, 8, 11 et 12 ou procédé selon l'une quelconque des revendications 9 à 13, où le milieu de culture cellulaire est supplémenté avec de l'ascorbate ou de l'albumine.
